# EUROPÄISCHE PATENTSCHRIFT

(11) **EP 3 131 605 B1**
(45) Veröffentlichungstag und Bekanntmachung des Hinweises auf die Patenterteilung: **01.08.2018**
(21) Anmeldenummer: 15716393.2
(22) Anmeldetag: 09.03.2015
(51) Int. Cl.: A61M 5/24, A61M 5/31, A61M 5/315, G01F 23/26, A61J 1/06

(54) **MEDIKAMENTENINJEKTOR MIT KAPAZITIVER FÜLLSTANDSMESSUNG UND BERÜHRUNGSSENSOR**
DRUG INJECTION DEVICE WITH CAPACITIVE FILL LEVEL DETERMINATION AND TOUCH SENSOR
INJECTEUR DE MÉDICAMENTS AVEC CAPTEUR CAPACITIF POUR DÉTERMINER NIVEAU DE REMPLISSAGE DU RÉSERVOIR ET CAPTEUR DE CONTACT

(30) Priorität: 15.04.2014 AT 502832014; 09.07.2014 AT 504772014
(43) Veröffentlichungstag der Anmeldung: 22.02.2017
(73) Patentinhaber: Seibersdorf Labor GmbH, 2444 Seibersdorf (AT); AIT Austrian Institute of Technology GmbH, 1220 Wien (AT)
(72) Erfinder: BAMMER, Manfred, 1220 Wien (AT); SCHMID, Gernot, 2833 Bromberg (AT); PUTZ, Otmar, 2833 Bromberg (AT)
(74) Vertreter: Wildhack & Jellinek
(86) Internationale Anmeldenummer: PCT/AT2015/050061
(87) Internationale Veröffentlichungsnummer: WO 2015/157785

(56) Entgegenhaltungen:
- WO-A1-2006/021295
- WO-A1-2013/138830
- WO-A1-2014/052997
- WO-A2-2007/107558
- US-A- 6 110 148

## Beschreibung

Die Erfindung betrifft eine Abgabevorrichtung zur Abgabe von Flüssigkeiten, insbesondere von flüssigen Medikamenten an Personen, gemäß dem Oberbegriff des Patentanspruches 1. Weiters betrifft die Erfindung eine Abdeckkappe gemäß dem Oberbegriff des Patentanspruchs 25. Schließlich betrifft die Erfindung ein Verfahren zur Bestimmung und Validierung des Füllstandes in einem Behälter gemäß dem Oberbegriff des Patentanspruches 32.

Die Erfindung kann insbesondere im Gesundheitswesen, beispielsweise in der Medizintechnik, Pharma- und Biotechnologie, Medizin und Pflege, Studien, usw. zur Überwachung der Abgabe von Medikamenten an Patienten eingesetzt werden.

Aus dem Stand der Technik, zum Beispiel der Patentanmeldung WO 2014/052997 A1,
sind verschiedene Vorrichtungen zur Abgabe von Flüssigkeiten bekannt, bei denen die Menge der abgegebenen Flüssigkeit bzw. die in der Abgabevorrichtung vorhandene Flüssigkeitsmenge kapazitiv bestimmt wird. Aufgabe der vorliegenden Erfindung ist es, Fehlfunktionen bei kapazitiven Füllstandsdetektionen wirksam zu erkennen und eine Invalidierung von kapazitiven Füllstands-Messergebnissen zu ermöglichen. Weiters ist es Aufgabe der Erfindung, möglichst gute und zuverlässige Resultate zu erhalten.

Die Erfindung löst diese Aufgabe bei einer Abgabevorrichtung der eingangs genannten Art mit dem kennzeichnenden Merkmal des Patentanspruches 1.

Zudem löst die Erfindung die Aufgabe bei einer Abdeckkappe der eingangs genannten Art mit dem Merkmal des Kennzeichens des Patentanspruches 25.

Zudem löst die Erfindung die Aufgabe bei einem Verfahren der eingangs genannten Art mit dem Merkmal des Kennzeichens des Patentanspruches 32.

Erfindungsgemäß sind bei einer Abgabevorrichtung zur Abgabe von Flüssigkeiten, insbesondere von flüssigen Medikamenten an Personen, vorgesehen:
- ein mit der Flüssigkeit gefüllter Behälter, der an einem Ende eine Öffnung zur Abgabe der Flüssigkeit aufweist, sowie
- zumindest ein Paar von im Außenbereich, insbesondere an der Wand, des Behälters einander gegenüberliegend angeordneten kapazitiven Messelektroden zur Bestimmung der Permittivität des jeweiligen Mediums im Zwischenbereich zwischen den Messelektroden. Erfindungsgemäß wird eine stabile und genaue Ermittlung des Füllstands der Flüssigkeit im Behälter erzielt.

Vorteilhafterweise kann vorgesehen sein, dass die Messelektroden von einer um den Behälter angeordneten Abschirmung umgeben sind. Störungen, die durch das Berühren des Behälters während des Messvorganges aufgrund kapazitiver Effekte verursacht werden, werden mit der Erfindung wirksam vermieden. Insbesondere kann durch die erfindungsgemäße Maßnahme vermieden werden, dass eine Berührung der Messelektroden durch die Hände eines Menschen oder eine Verfälschung des elektrischen Feldes im Bereich der Messelektroden durch die Hände eines Menschen oder anderer elektrisch leitfähiger Objekte zu Änderungen des Füllstandsmesswertes führen.

Ein vorteilhafter Schutz der Messelektroden kann durch eine die Messelektroden und den Behälter mantelförmig umgebende Abdeckkappe erzielt werden.

Um gleichzeitig einen mechanischen und elektrisch wirksamen Schutz der Messelektroden zu erzielen, kann vorgesehen sein, dass die Abschirmung in der Abdeckkappe integriert oder auf der Oberfläche der Abdeckkappe aufgebracht ist.

Alternativ kann zum selben Zweck vorgesehen sein, dass die Wand der Abdeckkappe die Messelektroden umgibt und dass die Außenwand oder Außenoberfläche der Abdeckkappe einen Abstand von mindestens 5 mm, insbesondere von mindestens 1cm, von den Messelektroden aufweist und/oder einen Zugriff und eine Berührung auf die Messelektroden verhindert.

Vorteilhafterweise kann die Abschirmung als mit Leiterbahnen aus elektrisch leitfähigem Material beschichtete Folie ausgebildet sein. In einer bevorzugten Ausführungsform ist diese Folie um den Behälter herum angeordnet oder gewickelt und/oder umgibt diesen. Eine solche Abschirmung verhindert die Verfälschung von Messergebnissen besonders vorteilhaft.

Um gute Messergebnisse zu erzielen, kann vorgesehen sein, dass der Bereich zwischen der Flüssigkeit und den Messelektroden frei von der Abschirmung ist.

Um eine Verschlechterung der Messergebnisse durch Einflüsse der Abschirmung zu vermeiden, kann vorgesehen sein, dass die Abschirmung von den Messelektroden in radialer Richtung beabstandet ist.

Um eine verbesserte Abschirmung zu erreichen und gleichzeitig eine Befestigung eines Halbleiterchips und einer Antenne im Bereich der Abschirmung oder auf der Folie zu ermöglichen, kann vorgesehen sein, dass die Abschirmung als mit Leitern in Form von Leiterbahnen beschichtete, insbesondere um den Behälter gewickelte, Folie ausgebildet ist, wobei vorzugsweise auf der Folie eine Kapazitätsmesschaltung, eine Recheneinheit und ein Kommunikationscontroller, insbesondere in Form eines Halbleiterchips, sowie eine Antenne aufgebracht sind.

Um eine die Funkkommunikation mit der Antenne beeinträchtigende Veränderung des von einem externen Datenkommunikationsgerätes erzeugten elektromagnetischen Feldes zu vermeiden und gleichzeitig eine gute elektrische Abschirmung der Messelektroden zu ermöglichen, kann vorgesehen sein, dass die Leiter schleifenfrei und/oder frei von geschlossenen Leiterschleifen ausgebildet sind.

Für eine gute Abschirmwirkung kann vorgesehen sein, dass die Leiter eine Breite von höchstens 5 mm, insbesondere von zwischen 0,1 mm und 3 mm, und eine Dicke von höchstens 3 mm, insbesondere von zwischen 10 µm und 150 µm, aufweisen.

Eine elektronisch vorteilhafte Umsetzung der Erfassung des Füllstandes im Behälter sieht vor, dass auf der Folie ein Kommunikationscontroller und/oder eine Anzahl von Kapazitätsmesseinrichtungen zur Ermittlung der Kapazität zwischen den Messelektroden und/oder eine Recheneinheit und/oder eine Antenne zur Übertragung von mit den Messelektroden ermittelten Messwerten oder daraus abgeleiteten Werten, insbesondere von Füllstandswerten, angeordnet sind, und dass diese auf der Folie angeordneten Einheiten vorzugsweise in einem gemeinsamen Gehäuse eines Halbleiterchips integriert sind.

Eine besonders vorteilhafte Ausgestaltung einer Abschirmung, die gleichzeitig zur Berührungsdetektion verwendet werden kann und zudem eine Funkkommunikation mit einer auf die Abschirmung aufgebrachten Antenne ermöglicht, sieht vor, dass auf der Folie drei separate Leiter ausgebildet sind, wobei der erste und der zweite Leiter als ineinandergreifende Kammleiter ausgebildet sind und der dritte Leiter mäanderförmig ausgebildet zwischen den beiden Kammleitern liegt.

Eine bevorzugte Maßnahme zur Bestimmung des Füllstandes der Flüssigkeit im Inneren des Behälters sieht vor, dass die beiden gegenüberliegenden Messelektroden an eine Kapazitätsmesseinrichtung angeschlossen sind.

Zur einfachen Ermittlung des Füllstandes kann vorgesehen sein, dass der von der Kapazitätsmesseinrichtung ermittelte Kapazitätswert einer Recheneinheit zugeführt ist, die aufgrund des ermittelten Kapazitätswerts mittels einer vorgegebenen abgespeicherten Kalibrierfunktion den Füllstand der Flüssigkeit im Behälter bestimmt und an ihrem Ausgang zur Verfügung hält.

Eine besonders wirksame Abschirmung mit guter Abschirmungswirkung kann erzielt werden, indem einer der drei Leiter, insbesondere der als Kammleiter ausgebildete zweite Leiter, mit dem Massenanschluss der Kapazitätsmesseinrichtung verbunden ist.

Um Berührungen oder Verfälschungen der Kapazitätsmessung vorteilhaft feststellen zu können, kann ein außerhalb oder im Bereich der Abschirmung angeordneter, insbesondere kapazitiver, Berührungssensor vorgesehen sein.

Eine produktionstechnisch einfach zu fertigende Variante sieht vor, dass der Berührungssensor den ersten Kammleiter und den mäanderförmigen Leiter der Abschirmung als Sensorelektroden umfasst.

Hierbei kann zur Detektion von Berührungen vorgesehen sein, dass die Sensorelektroden des Berührungssensors an eine weitere Kapazitätsmesseinrichtung angeschlossen sind, und dass vorzugsweise der von der weiteren Kapazitätsmesseinrichtung ermittelte weitere Kapazitätswert der Recheneinheit zugeführt ist, und die Recheneinheit für den Fall, dass der ermittelte weitere Kapazitätswert einen vorgegebenen Schwellenwert übersteigt, die Weiterleitung des von ihr ermittelten Füllstands unterdrückt oder als ungültig kennzeichnet.

Ein vorteilhafter Behälter zur Aufnahme von Flüssigkeiten, der einfach entleert werden kann und dessen Füllstand einfach festgestellt werden kann, sieht vor, dass der Behälter ein Innenvolumen aufweist, das abgesehen vom Bereich der Öffnung einen konstanten Innenquerschnitt aufweist,
wobei ein den Behälter und die darin befindliche Flüssigkeit abschließender und abdichtender Kolben vorgesehen ist, dessen Außenquerschnitt dem Querschnitt des Innenvolumens des Behälters entspricht und der im Inneren des Behälters verschiebbar angeordnet ist, sodass bei Vorschub des Kolbens zur Öffnung hin die Flüssigkeit durch die Öffnung aus dem Behälter abgegeben wird.

Zur genaueren Bestimmung des Füllstandes kann vorgesehen sein, dass am Behälter eine Vielzahl von Paaren von zusätzlichen Messelektroden angeordnet sind, wobei insbesondere für jedes Paar von zusätzlichen Messelektroden jeweils eine zusätzliche dem Paar von zusätzlichen Messelektroden nachgeschaltete Kapazitätsmesseinrichtung vorgesehen ist, die den ermittelten Kapazitätswert an die Recheneinheit abgibt.

Eine vorteilhafte Elektrodenanordnung, die eine genaue Füllstandsbestimmung ermöglicht, sieht vor, dass die jeweils einander paarweise zugeordneten Messelektroden einander in Umfangsrichtung des Behälters, insbesondere diametral, gegenüberliegen und insbesondere in Richtung des Vorschubs des Kolbens auf derselben Position oder Höhe liegen.

Hierbei kann zusätzlich zur Verbesserung der Detektionsgenauigkeit vorgesehen sein, dass jeweils benachbarte Paare von Messelektroden in Richtung des Vorschubs des Kolbens beabstandet angeordnet sind und/oder
dass die Breite der Messelektroden in Richtung des Vorschubs des Kolbens der Breite des Kolbens in dessen Vorschubrichtung entspricht.

Bevorzugte Ausführungen der Messelektroden mit einem einfachen Aufbau sehen vor,
- dass die Messelektroden flächenhaft auf der Außenoberfläche des Behälters angeordnet sind und insbesondere die Form eines Rechtecks, eines Dreiecks, eines Trapezes oder eines Parallelogramms aufweisen, und/oder
- dass je zwei der einander paarweise zugeordneten Messelektroden durch zwei ineinandergreifende Kammleiter ausgebildet sind, die im Außenbereich, insbesondere an der Außenwand, des Behälters angeordnet sind.

Um einen einfachen Austausch der Behälter zu ermöglichen, kann vorgesehen sein, dass außerhalb des Behälters zwischen dem Behälter und der Abschirmung ein Träger angeordnet ist, auf dem die Messelektroden angeordnet sind, wobei der Träger vorzugsweise am Behälter anliegt und/oder dass die Messelektroden auf der am Behälter anliegenden Wand des Trägers angeordnet sind, wobei insbesondere ein Teil des Gehäuses der Abgabevorrichtung als Träger ausgebildet ist oder der Träger mit dem Gehäuse verbunden ist.

Vorteilhafterweise kann der ermittelte Füllstand an ein externes Kommunikationsgerät übermittelt werden. Hierbei kann vorgesehen sein, dass an die Recheneinheit ein Kommunikationscontroller mit einer ihm nachgeschalteten Antenne angeschlossen ist. Vorteilhafterweise kann für eine platzsparendere Anordnung vorgesehen sein, dass die Antenne im Außenbereich der Abschirmung oder unmittelbar auf der Abschirmung, jedoch nicht elektrisch leitend mit dieser verbunden, angeordnet ist.

Eine einfach aufgebaute Abgabevorrichtung, die eine wiederverwendbare Abdeckkappe zur Füllstandsmessung und gegebenenfalls -anzeige aufweist, sieht vor, dass die Abdeckkappe eine Anzahl von elektrischen Kontakten aufweist, die jeweils mit einer der Messelektroden des Behälters, insbesondere über auf der Abdeckkappe angeordnete Anschlusskontakte, elektrisch leitend verbunden sind.

Eine produktionstechnisch einfache Lösung sieht vor, dass die Abschirmung und/oder die Leiter, die Kapazitätsmesseinrichtungen und/oder die weitere Kapazitätsmesseinrichtung und/oder die Recheneinheit und/oder der Kommunikationscontroller und/oder die Antenne und/oder eine Spannungsversorgung in die Abdeckkappe integriert sind.

Um einen guten Halt zwischen Abdeckkappe und dem Körper der Abgabevorrichtung zu ermöglichen, kann vorgesehen sein,
- dass die Abdeckkappe im Bereich der Öffnung des Behälters eine durchgehende Ausnehmung zur Abgabe von Flüssigkeit durch die Abdeckkappe hindurch aufweist, durch die gegebenenfalls eine mit der Flüssigkeit in Kontakt stehende Injektionsnadel hindurchgeführt ist, und/oder
- dass die Abdeckkappe den Behälter, und insbesondere eine mit der Flüssigkeit in Kontakt stehende Injektionsnadel, umgibt, und/oder
- dass die Abdeckkappe mit dem Behälter und/oder mit einem an den Behälter anschließenden Gehäuseteil, insbesondere mehrfach lösbar, verbindbar ist, und/oder
- dass die Abdeckkappe in Form einer Hülse ausgebildet, die an dem Ende der Injektionsnadel offen ist.

Um den Behälter während der Abgabe und/oder Messung beobachten zu können und etwaige Trübungen der im Behälter befindlichen Flüssigkeit oder Fremdkörper erkennen zu können, kann vorgesehen sein, dass im Trägerkörper ein Sichtfenster vorgesehen ist, durch die die Flüssigkeit sichtbar ist, wobei gegebenenfalls die Abdeckkappe im Bereich des Sichtfenster ein weiteres Sichtfenster aufweist, durch die die Flüssigkeit sichtbar ist, und/oder
dass eine Anzeigeeinheit im Außenbereich der Abdeckkappe vorgesehen ist, die insbesondere der Füllstand der Flüssigkeit im Inneren des Behälters anzeigt.

Weiters betrifft die Erfindung eine Abdeckkappe zum Abdecken und Abschirmen eines mit Flüssigkeit gefüllten Behälters, der eine Anzahl von Messelektroden aufweist, umfassend
- auf der Abdeckkappe, insbesondere innenseitig, angeordnete Anschlusskontakte zur elektrischen Kontaktierung der Messelektroden,
- zumindest eine mit den Anschlusskontakten verbundene Kapazitätsmesseinrichtung. Mit einer solchen wiederverwendbaren Abdeckkappe kann eine mehrfache Messung unterschiedlicher Behälter in unterschiedlichen Trägerkörpern vorgenommen werden. Insbesondere bei Sets mit mehreren Behältern kann eine mehrfache Realisierung einer Messeinrichtung auf allen Trägerkörpern verhindert werden.

Eine besonders vorteilhafte Anordnung sieht eine im Inneren der Abdeckkappe oder auf ihrer Oberfläche verlaufenden Abschirmung vor, die insbesondere mit einer weiteren Kapazitätsmesseinrichtung verbunden ist.

Eine vorteilhafte Abdeckkappe, mit der eine automatische Weiterverarbeitung von Daten unmittelbar auf der Abdeckkappe möglich ist, sieht folgendes vor:
eine der Kapazitätsmesseinrichtung oder den Kapazitätsmesseinrichtungen und/oder der weiteren Kapazitätsmesseinrichtung nachgeschalteten Recheneinheit, sowie gegebenenfalls einen der Recheneinheit nachgeschalteten Kommunikationscontroller, an den insbesondere eine Antenne angeschlossen ist.

Vorteilhaft kann eine auf oder in der Abdeckkappe angeordnete Spannungsversorgung vorgesehen sein, die an die Kapazitätsmesseinrichtung, sowie gegebenenfalls an die weitere Kapazitätsmesseinrichtung und/oder die Recheneinheit und/oder den Kommunikationscontroller angeschlossen ist und diese mit elektrischer Energie versorgt.

Um eine Abgabe von Flüssigkeit aus dem Behälter vorteilhaft zu ermöglichen, kann vorgesehen sein, dass eine, insbesondere im Bereich der Öffnung des Behälters befindliche, durch die Abdeckkappe durchgehende Ausnehmung zur Abgabe von Flüssigkeit durch die Abdeckkappe hindurch vorgesehen ist, und/oder dass die Abdeckkappe in Form einer Hülse ausgebildet, die nach einer Seite offen ist.

Um eine mehrfache Verwendung der Abdeckkappe sicherzustellen, kann vorgesehen sein, dass die Abdeckkappe zur, insbesondere mehrfach, lösbaren Verbindung mit dem Behälter oder einem mit diesem verbundenen Gehäuseteil ausgebildet ist.

Um den Behälter während der Abgabe und/oder Messung beobachten zu können und etwaige Trübungen der im Behälter befindlichen Flüssigkeit oder Fremdkörper erkennen zu können, kann vorgesehen sein, dass ein Sichtfenster vorgesehen ist, durch die die im Inneren der Abdeckkappe und des Behälters befindliche Flüssigkeit von außen sichtbar ist.

Alternativ oder zusätzlich kann auch vorgesehen sein, dass eine Anzeigeeinheit im Außenbereich der Abdeckkappe vorgesehen ist, die insbesondere der Füllstand der Flüssigkeit im Inneren des Behälters anzeigt.

Weiters betrifft die Erfindung ein Verfahren zur Bestimmung und Validierung des Füllstandes in einem Behälter, der insbesondere in einer erfindungsgemäßen Abgabevorrichtung angeordnet ist, wobei zumindest ein Paar von im Außenbereich des Behälters einander gegenüberliegend angeordneten, insbesondere mit einer äußeren Abschirmung versehenen, Messelektroden zur Kapazitätsmessung vorgesehen ist,
wobei die Kapazität zwischen den beiden Messelektroden ermittelt wird und aufgrund der ermittelten Kapazität gemäß einer vorgegebenen Kalibrierfunktion ein Füllstandswert bestimmt wird.

Erfindungsgemäß ist bei einem solchen Verfahren vorgesehen,
- dass eine weitere Kapazität mit im Außenbereich der Messelektroden im Bereich der Abschirmung, insbesondere auf der Abschirmung, angeordneten Leitern ermittelt wird,
- dass die weitere Kapazität mit einem Schwellenwert verglichen wird, und
- dass der Füllstandswert nur dann als gültig angesehen wird, wenn die weitere Kapazität unterhalb des Schwellenwerts liegt.

Mit einem solchen Verfahren kann einfach überprüft werden, ob der ermittelte Füllstandswert dadurch verfälscht wurde, dass eine Person die Messelektroden oder die Abschirmung im Bereich der Messelektroden berührt hat oder den Messelektroden hinreichend nahe gekommen ist, um eine Verfälschung zu verursachen.

Zur genauen Bestimmung des Füllstandes kann vorgesehen sein, dass der Füllstandwert und/oder eine Aussage über die Gültigkeit des Füllstandswertes durch codierte elektromagnetischer Datenübertragung, insbesondere durch Lastmodulation, an ein externes Datenkommunikationsgerät übertragen wird.

Zum selben Zweck kann vorgesehen sein, dass jeweils die Kapazitäten einer Vielzahl von, insbesondere drei, Paaren von Messelektroden, wobei die einander paarweise zugeordneten Messelektroden einander im Außenbereich des Behälters gegenüberliegen, ermittelt werden und der Füllstandswert aufgrund der Kapazitäten ermittelt wird.

Eine besonders genaue Detektion wird ermöglicht, indem
a) für eine Anzahl von Füllständen jeweils Referenzvektoren umfassend die Kapazitäten zwischen den einzelnen Paaren von Messelektroden als Komponenten zur Verfügung gestellt werden, und
b) jedem dieser Vektoren der jeweilige Füllstand zugeordnet wird,
c) ein Vektor umfassend die einzelnen ermittelten Kapazitäten ermittelt wird,
d) eine Anzahl von Referenzvektoren gesucht wird, die vom Vektor den geringsten, insbesondere euklidischen, Abstand aufweisen,
e) eine Interpolationsfunktion gebildet wird, die bei Anwendung auf die in Schritt b) aufgefundenen Referenzvektoren den jeweiligen, diesen Referenzvektoren zugeordneten Füllstand ergibt,
f) die Interpolationsfunktion auf den Vektor angewendet wird und das Ergebnis als Füllstand herangezogen wird.

Mehrere bevorzugte Ausführungsformen der Erfindung werden anhand der folgenden Zeichnungsfiguren näher erläutert.

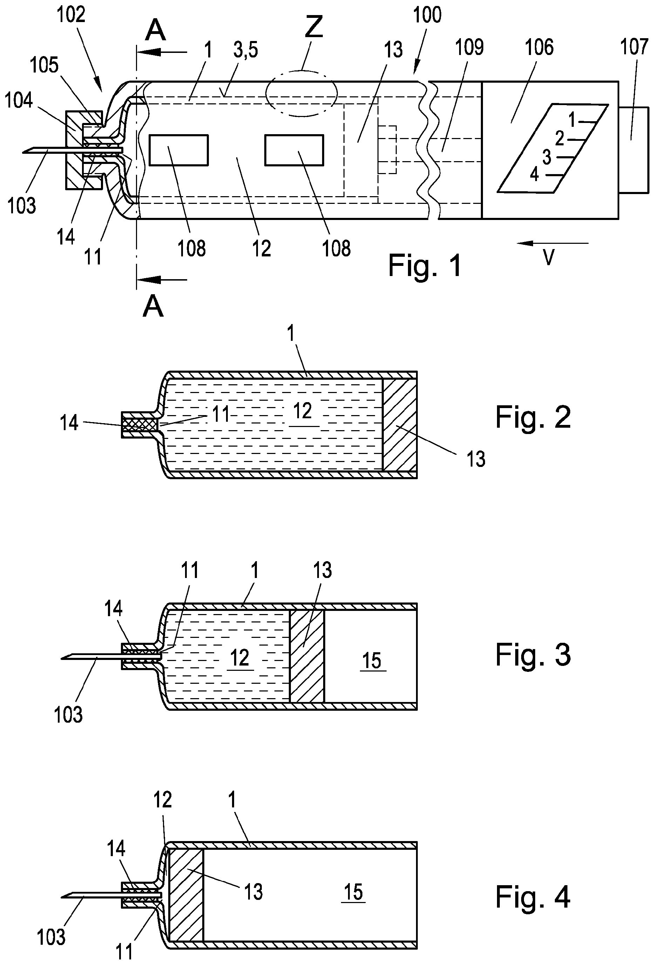

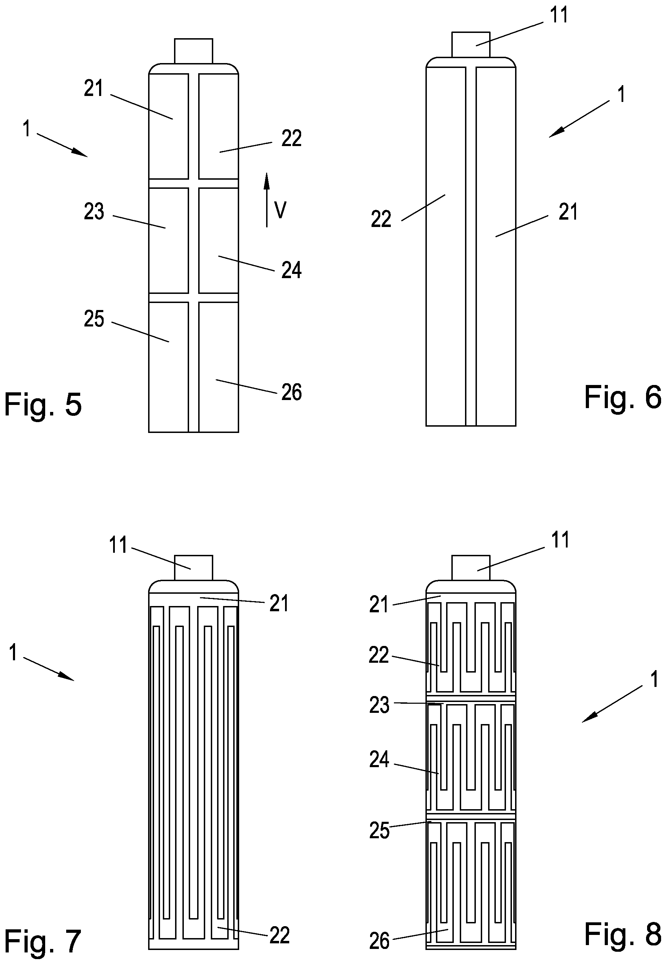

In **Fig. 1** ist eine erste Ausführungsform einer erfindungsgemäßen Abgabevorrichtung in Seitenansicht dargestellt. **Fig. 2** zeigt einen vollständig gefüllten Behälter in Form einer Ampulle in Seitenansicht. **Fig. 3** zeigt einen teilweise entleerten Behälter in Seitenansicht. **Fig. 4** zeigt einen vollständig entleerten Behälter in Seitenansicht. **Fig. 5** zeigt eine alternative Ausführungsform eines Behälters mit drei Paaren von Messelektroden. **Fig. 6** zeigt eine zweite Ausführungsform der Erfindung mit einem einzigen Paar von Messelektroden. **Fig. 7** zeigt eine weitere Ausführungsform der Erfindung mit einem Paar von kammförmig angeordneten Messelektroden. **Fig. 8** zeigt eine weitere Ausführungsform der Erfindung mit drei kammförmig angeordneten Paaren von Messelektroden.

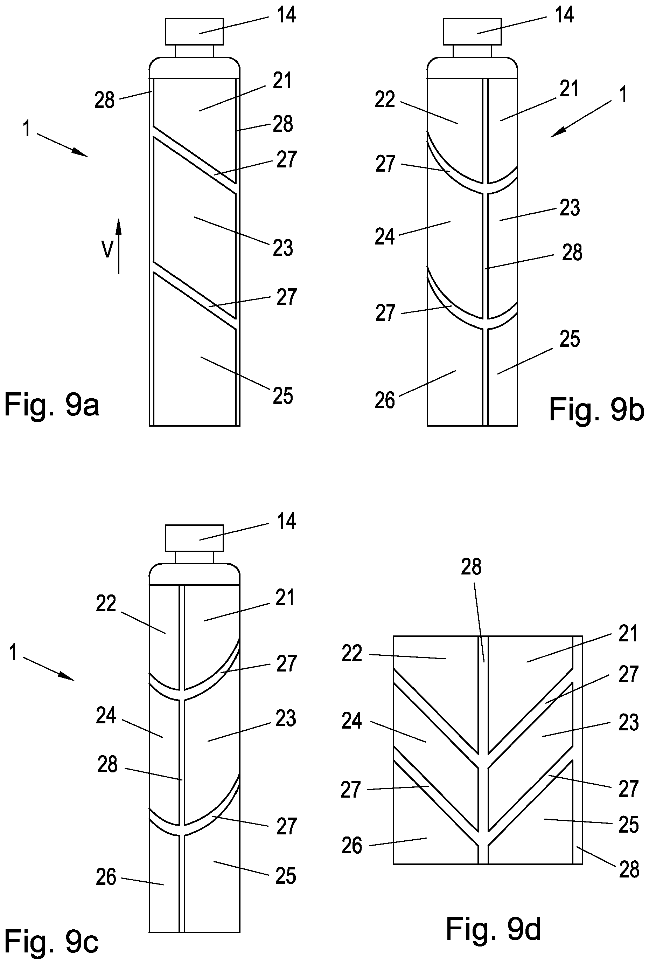

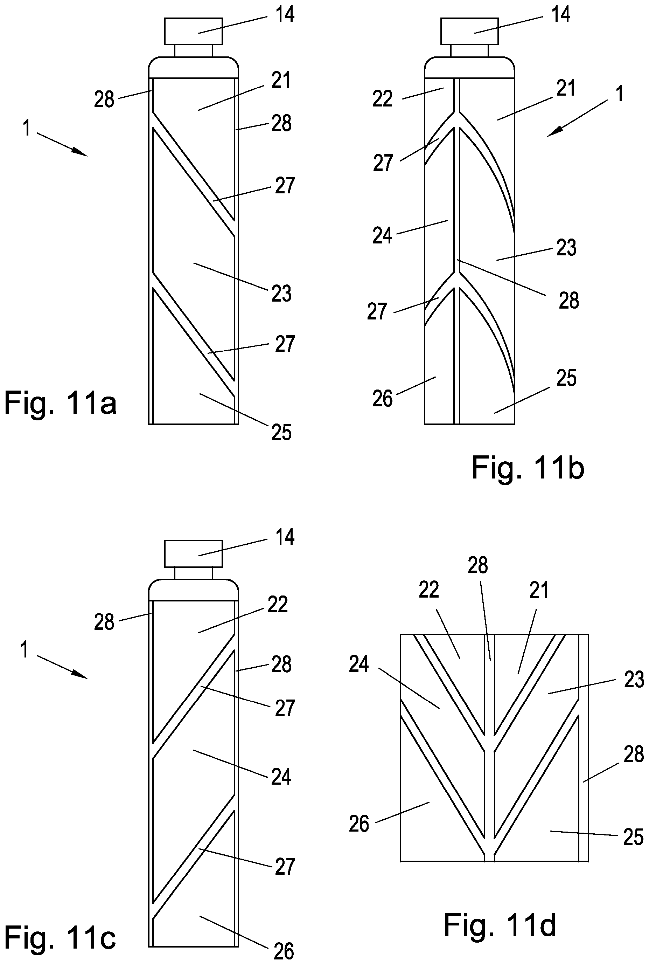

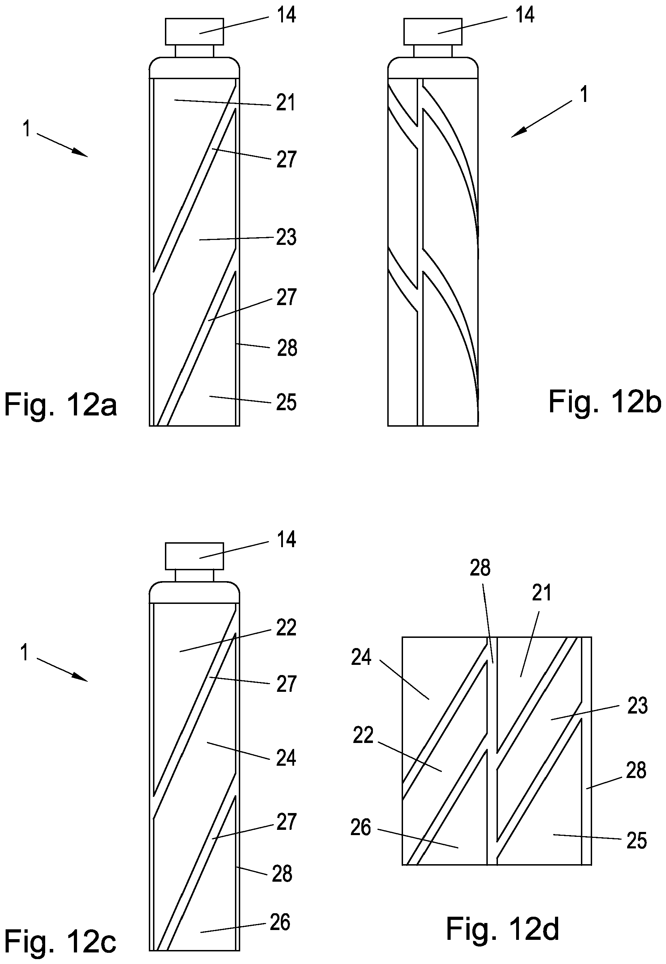

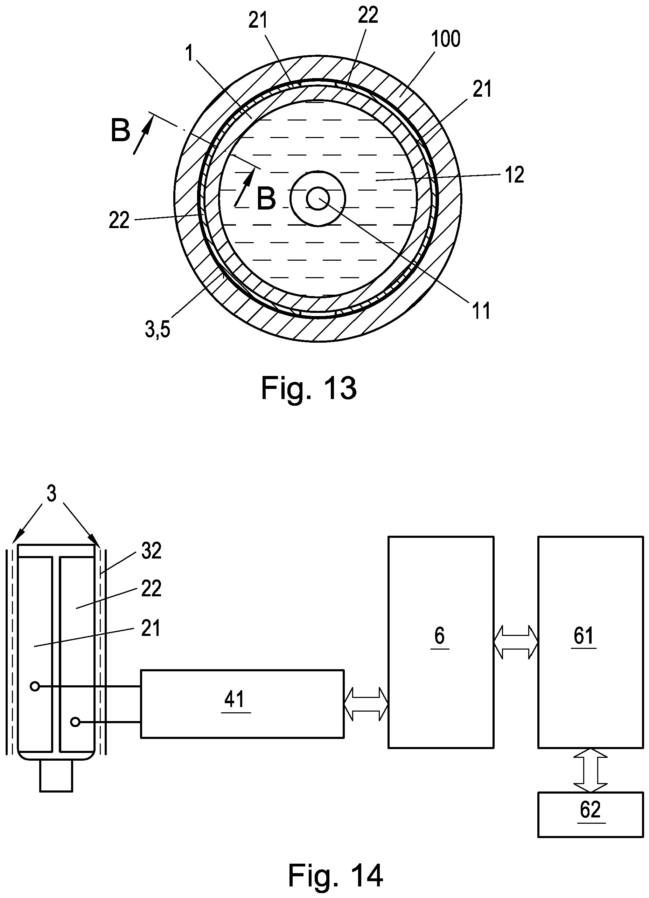

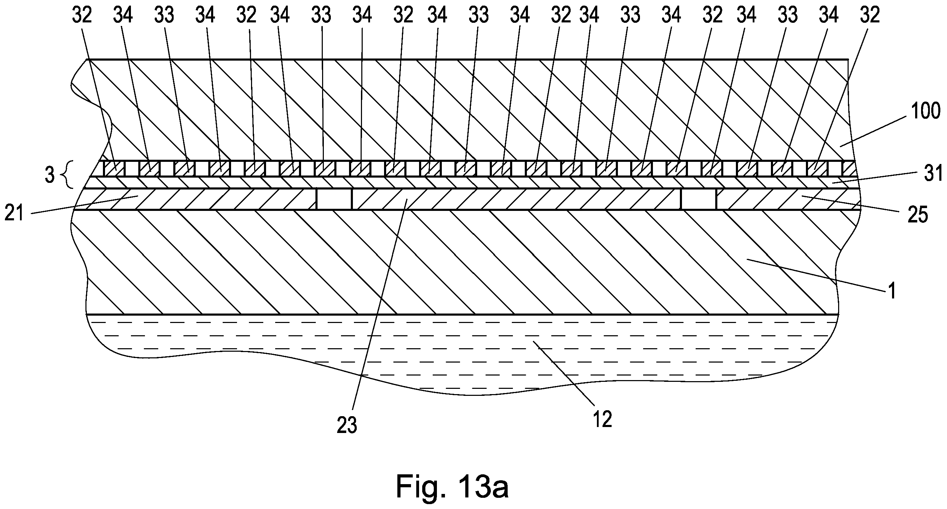

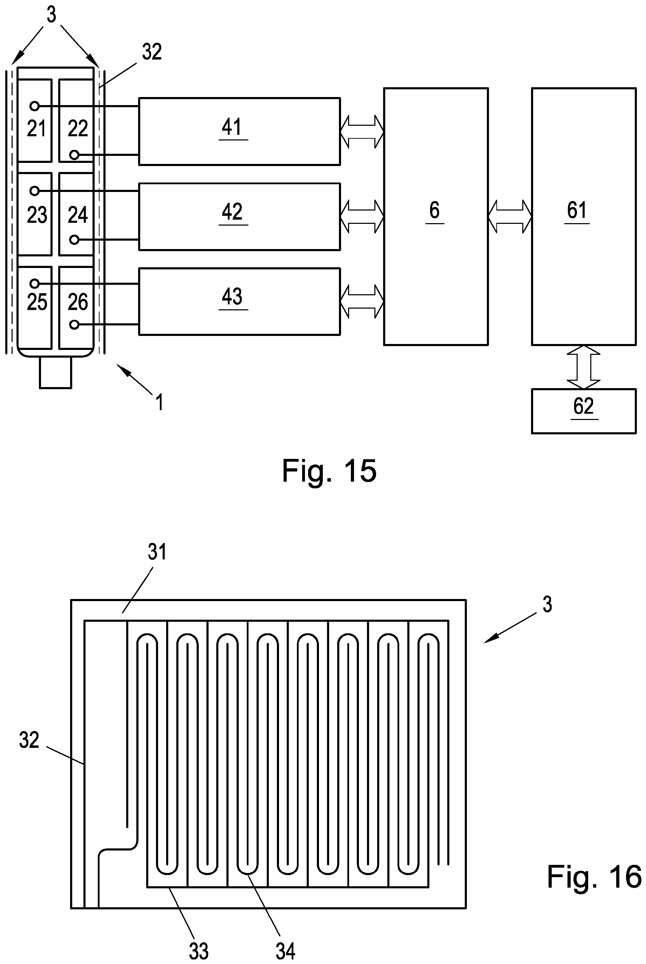

Die **Fig. 9a bis 12d** zeigen weitere Ausführungsformen von Behältern mit schräg verlaufenden Elektroden. **Fig. 13** zeigt ein Ausführungsbeispiel einer erfindungsgemäßen Vorrichtung im Querschnitt B-B. **Fig. 13a** zeigt ein Detail Z aus **Fig. 1****.** **Fig. 14** und **Fig. 15** zeigen zwei Einrichtungen zur Bestimmung des Füllstands innerhalb des Behälters sowie zur Übertragung des ermittelten Füllstands an ein externes Datenkommunikationsgerät.

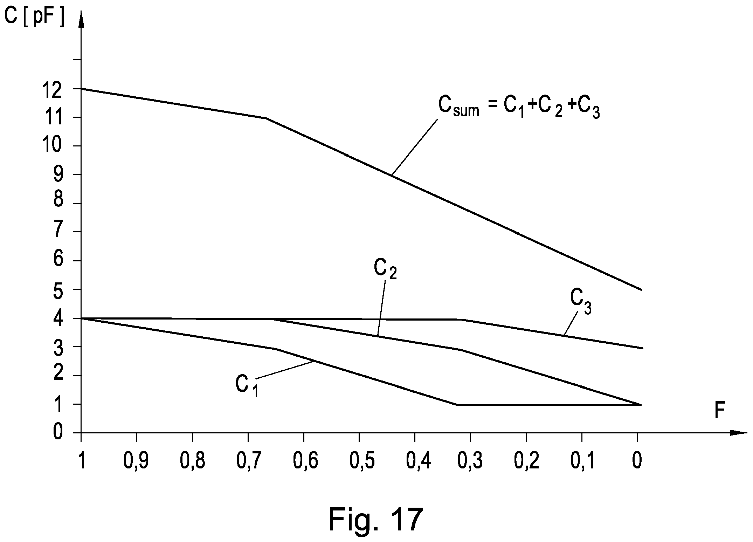

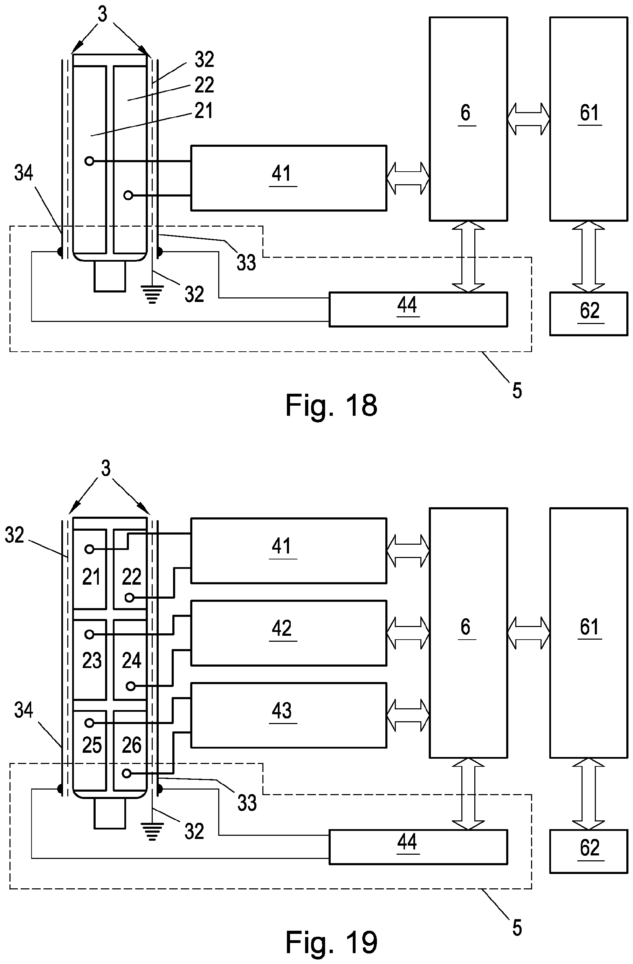

**Fig. 16** zeigt eine Abschirmung in Form einer Folie mit darauf angeordneten Leitern. **Fig. 17** zeigt den theoretischen Verlauf der einzelnen Teilkapazitäten während der Entleerung des Behälters bei der in **Fig. 15** dargestellten Ausführungsform. Die **Fig. 18 und 19** zeigen die in den **Fig. 14** **und** **15** dargestellten Ausführungsformen, wobei zusätzlich eine Berührungsdetektion vorgesehen ist.

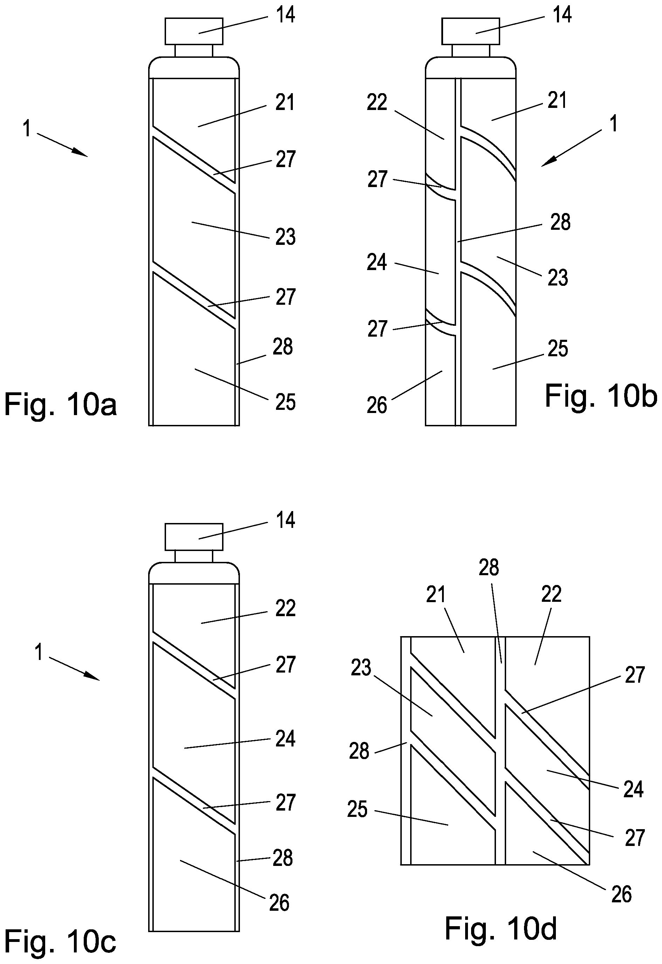

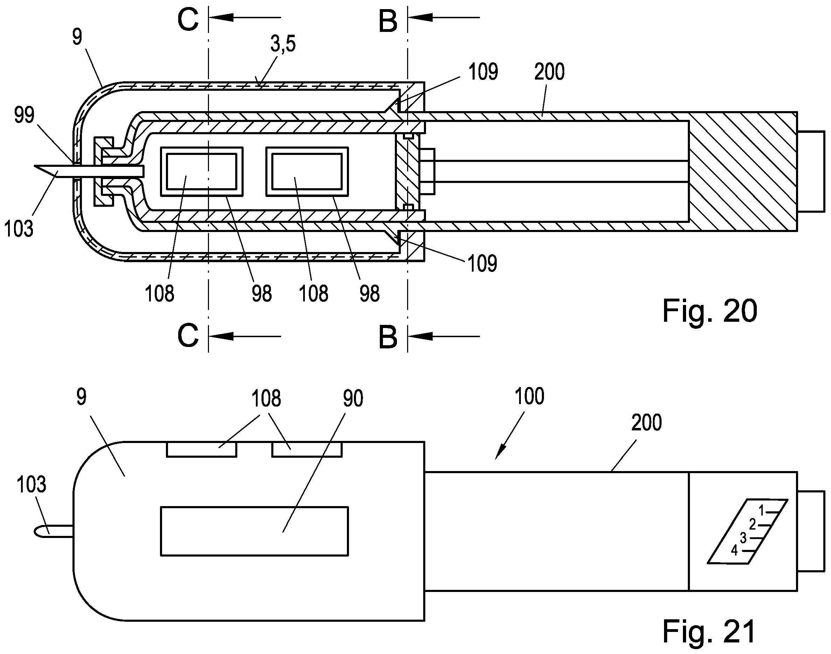

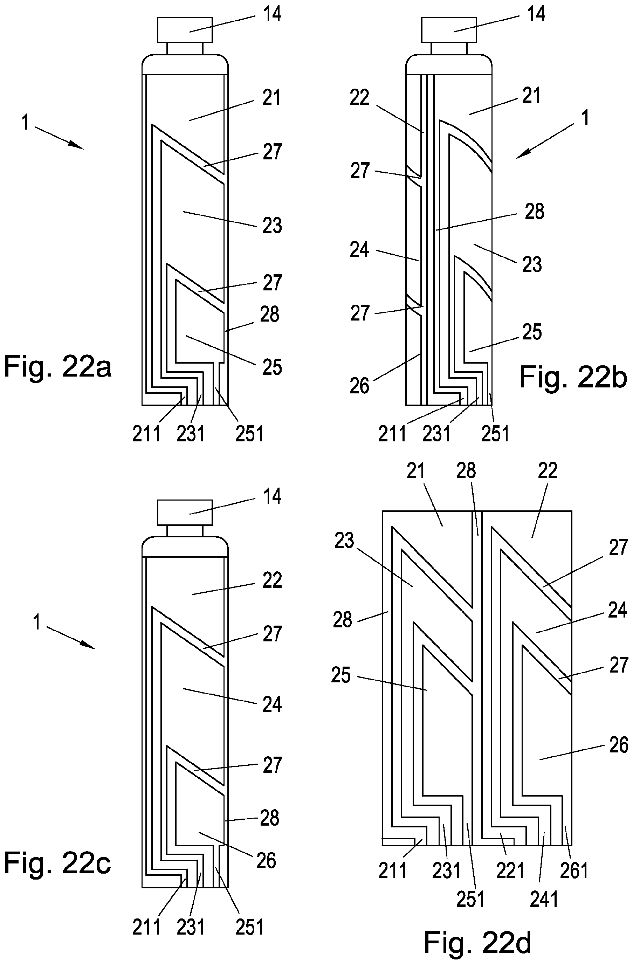

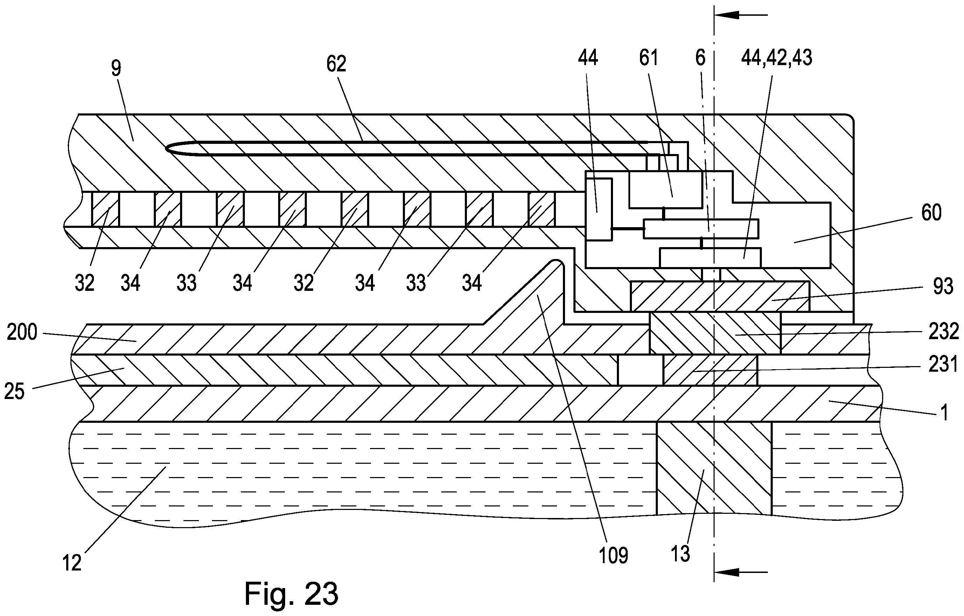

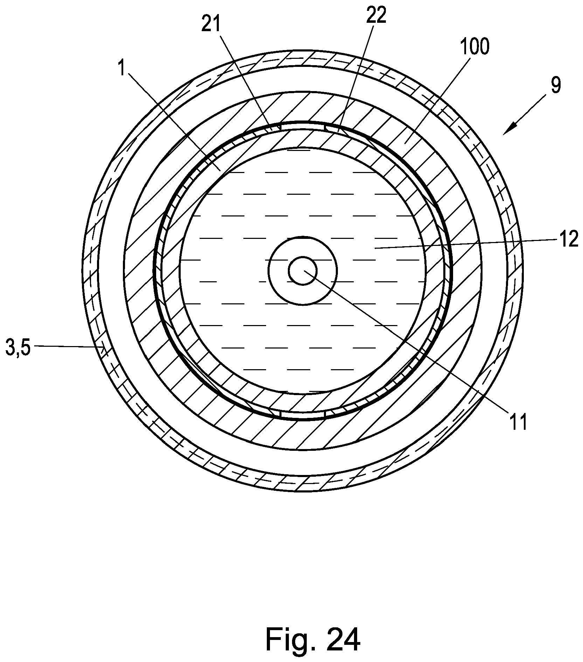

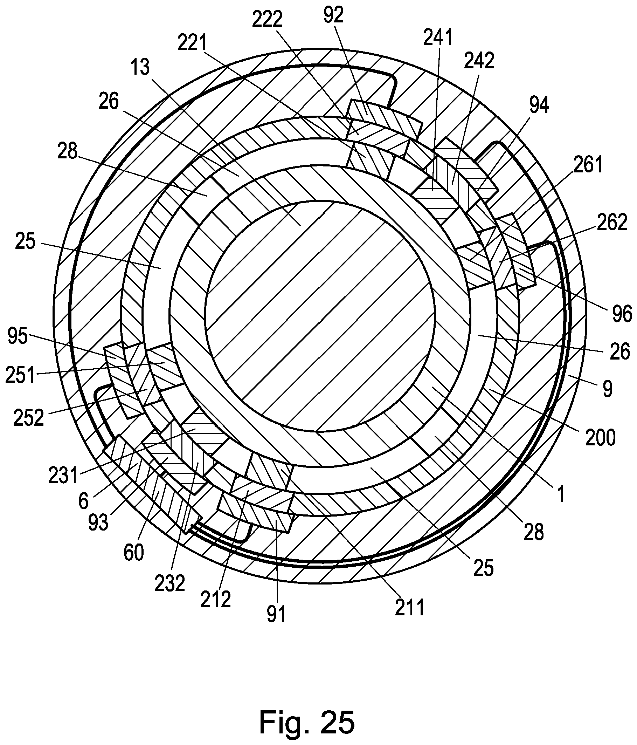

**Fig. 20** zeigt eine weitere Ausführungsform der Erfindung mit einer Abdeckkappe. **Fig. 21** zeigt eine Außenansicht der in **Fig. 20** dargestellten Ausführungsform. Die **Fig. 22** zeigt eine den **Fig. 10a****-d** entsprechende weitere Ausführungsform eines Behälters mit schräg verlaufenden Elektroden. **Fig. 23** zeigt ein Detail aus **Fig. 20****.** **Fig. 24** **und** **25** zeigen Schnitte durch die in **Fig. 20** dargestellte Ausführungsform der Erfindung.

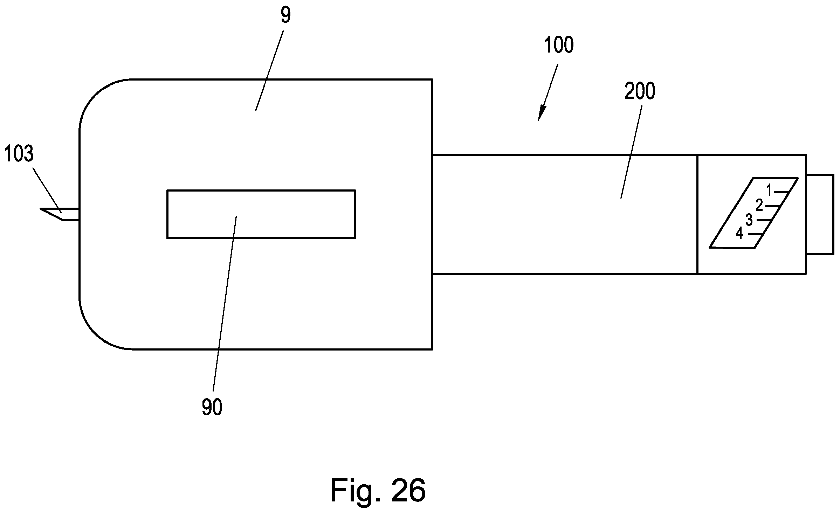

**Fig. 26** zeigt eine weitere Ausführungsform der Erfindung, die ohne Abschirmung auskommt.

In **Fig. 1** ist eine Ausführungsform einer erfindungsgemäßen Abgabevorrichtung 100 in Seitenansicht dargestellt. Die dargestellte Abgabevorrichtung 100 weist einen Behälter 1 auf, der mit einem flüssigen Medikament 12 gefüllt ist. Im vorliegenden Fall wird als flüssiges Medikament 12 Insulin verwendet, es ist jedoch auch möglich, andere flüssige Medikamente 12 wie etwa Hormonpräparate (z.B. Wachstumshormone, ...), Biopharmazeutika oder im Zuge therapeutischer Maßnahmen in der Fortpflanzungsmedizin verwendete Medikamente in den Behälter 1 zu füllen und anschließend auf dieselbe Art zu verabreichen.

Die Abgabevorrichtung 100 weist die Form eines Schreibstifts oder Kugelschreibers auf und kann von einem Patienten bei der Verabreichung der im Behälter 1 befindlichen Flüssigkeit 12 bequem in der Hand gehalten werden. Der Behälter 1 hat die Form einer Patrone oder Ampulle und befindet sich in einem Endbereich 102 der Abgabevorrichtung 100.

Der Behälter 1, der im Detail in **Fig. 2** dargestellt ist, weist an einem Ende, das in diesem Endbereich 102 der Abgabevorrichtung 100 liegt, eine Öffnung 11 zur Abgabe der Flüssigkeit 12 auf. Am gegenüberliegenden Ende weist der Behälter 1 einen Kolben 13 auf, der im Behälter 1 verschiebbar gelagert ist. Hierfür weist der Behälter 1 ein Innenvolumen auf, das abgesehen vom Bereich der Öffnung 11 einen konstanten Querschnitt aufweist. Der Kolben 13 verschließt den Behälter 1 von der der Öffnung 11 gegenüberliegenden Seite her, sodass die im Behälter 1 befindliche Flüssigkeit 12 dicht im Behälter 1 eingeschlossen ist und ausschließlich durch die Öffnung 11 entweichen kann. Im vorliegenden Ausführungsbeispiel weisen der Innenbereich des Behälters 1 sowie der Kolben 13 einen kreisförmigen Querschnitt auf und verfügen über eine im wesentlichen zylindrische Innenwand bzw. Außenwand. Wird der Kolben 13 in den Behälter 1 eingeschoben bzw. vorgeschoben, so kann die im Behälter 1 befindliche Flüssigkeit 12 durch die Öffnung 11 aus dem Behälter 1 entweichen. Bei Vorschub des Kolbens 13 in Richtung der Öffnung 11 wird die Flüssigkeit 12 durch die Öffnung 11 aus dem Behälter 1 abgegeben. Die Öffnung 11 des Behälters 1 ist jedoch vor dem Gebrauch, wie in Fig. 2 dargestellt, durch ein Versiegelungselement 14 verschlossen, sodass die Flüssigkeit 12 aus dem Behälter 1 nicht entweichen kann.

**Fig. 3** zeigt den in **Fig. 2** dargestellten Behälter 1, nachdem ein Teil der Flüssigkeit 12 durch die Öffnung 11 über eine Injektionsnadel 103 appliziert wurde.

**Fig. 4** zeigt den in **Fig. 2** dargestellten Behälter 1, nachdem die Flüssigkeit 12 aus dem Behälter 1 durch die Öffnung 11 über eine Injektionsnadel 103 vollständig entleert wurde. In den Darstellungen der **Fig. 3** und **Fig. 4** befindet sich der Kolben 13 in einer mittleren Position bzw. in Endstellung, d.h. der Behälter 1 ist teilweise **(****Fig. 3****)** bzw. vollständig **(****Fig. 4****)** entleert. Im Bereich 15 hinter dem Kolben 13 befindet sich Luft. Die Abgabevorrichtung 100 weist weiters im Bereich der Öffnung 11 des Behälters 1 eine Injektionsnadel 103 auf, die einerseits das Versiegelungselement 14 durchdringt und in das Innere des Behälters 1 ragt und andererseits von der Abgabevorrichtung 1 absteht.

Wie in **Fig. 1** dargestellt, ist die Injektionsnadel 103 in diesem Ausführungsbeispiel mit einem Gehäuse 104 verbunden, das auf die Abgabevorrichtung 100 aufgeschraubt ist. Die Abgabevorrichtung 100 weist ein Außengewinde 105 auf, das an ein gegengleich geformtes passendes Innengewinde des Gehäuses 104 angepasst ist. Wird der Kolben 13, wie in **Fig. 3** dargestellt, in Richtung der Öffnung 11 verschoben, so kann die im Inneren des Behälters 1 befindliche Flüssigkeit durch die Öffnung 11 und die Injektionsnadel 103 dem jeweiligen Patienten verabreicht werden. Das Gehäuse der Abgabevorrichtung 100 weist zwei Sichtöffnungen 108 auf, um den Füllstand F der im Behälter 1 befindlichen restlichen Flüssigkeit 12 visuell bestimmen oder etwaige Trübungen oder Fremdkörper in der Flüssigkeit 12 erkennen zu können.

Darüber hinaus weist die Abgabevorrichtung 100 **(****Fig. 1****)** eine Einstelleinheit 106 auf, mit der ein bestimmter Vorschub des Kolbens 13 und - damit korrespondierend - eine bestimmte Menge der abzugebenden Flüssigkeit 12 voreingestellt werden kann. Nach der Einstellung der Menge der abzugebenden Flüssigkeit 12 wird mittels Druckbetätigung durch den Patienten auf eine Betätigungseinheit 107 ein Vorschubelement 109 gegen den Kolben 13 des Behälters 1 gedrückt. Der Kolben 13 wird in den Behälter 1 geschoben und die im Behälter 1 befindliche Flüssigkeit 12 wird über die Injektionsnadel 103 an den Patienten verabreicht. Das Vorschubelement 109 ist gegen ein Zurücksetzen entgegen der Vorschubrichtung V des Kolbens 13, d.h. von der Öffnung 11 weg, gesichert, sodass der Kolben 13 nur noch weiter in Richtung der Öffnung 11 bewegt werden kann.

Besonders vorteilhaft kann die Verwendung eines Behälters 1 mit drei Paaren von Messelektroden sein, wie in **Fig. 5** dargestellt. Der Behälter 1 weist, wie in **Fig. 5** dargestellt, drei Paare von Messelektroden 21-26 auf. Sämtliche Messelektroden 21-26 sind im Außenbereich des Behälters 1, im vorliegenden Fall auf der Außenwand des Behälters 1 angeordnet. In diesem bevorzugten Ausführungsbeispiel der Erfindung liegen je zwei einander zugeordnete Messelektroden 21-26 einander in Umfangsrichtung beabstandet an der Außenwand des Behälters 1 gegenüber. Die einzelnen Paare einander zugeordneter Messelektroden 21-26 sind zueinander in Vorschubrichtung V des Kolbens 13 beabstandet. Die Messelektroden 25, 26 des dritten Paars liegen von der Öffnung 11 des Behälters 1 am weitesten entfernt. Die Messelektroden 21, 22 des ersten Paars liegen der Öffnung 11 am nächsten. Die Messelektroden 23, 24 des zweiten Elektrodenpaars liegen - in Vorschubrichtung V des Kolbens 13 gesehen - zwischen den Messelektroden 21, 22; 25, 26 des ersten und des dritten Paars. Die Messelektroden 21-26 liegen in einem Bereich der Außenwand des Behälters 1 flächig an dieser an. In dem in **Fig. 5** dargestellten Ausführungsbeispiel weisen die Messelektroden 21-26 eine rechteckige Form auf. Die Messelektroden 21-26 erstrecken sich über den gesamten Vorschubbereich des Kolbens 13. Werden mehrere Elektrodenpaare verwendet, kann es vorteilhaft sein, wenn die Ausdehnung eines Elektrodenpaars in Vorschubrichtung V des Kolbens 13 der Ausdehnung des Kolbens 13 in seiner Vorschubrichtung V entspricht.

Alternativ können jedoch auch andere Elektrodenformen, etwa kreisförmige oder kammartige Elektrodenformen für die Messelektroden 21-26 verwendet werden. Die Verwendung mehrerer Paare von Messelektroden 21-26 ist im Sinne einer genauen Messung des Flüssigkeitsinhalts oder Flüssigkeitsstands in länglichen Behältern 1 zwar grundsätzlich vorteilhaft, aber gerade bei kurzen oder kompakten Behältern 1 nicht erforderlich. In einem in **Fig. 6** dargestellten alternativen Ausführungsbeispiel eines Behälters 1 ist lediglich ein einziges Paar von Messelektroden 21, 22 vorgesehen, die länglich und sich über den gesamten Vorschubbereich erstreckend ausgebildet sind. Die beiden Messelektroden 21, 22 liegen einander umfangsseitig auf derselben Höhe oder Position hinsichtlich der Vorschubrichtung V des Kolbens 13 gegenüber.

Darüber hinaus ist es auch möglich, unterschiedliche Formen von Messelektroden 21-26 zu verwenden. Eine vorteilhafte Ausführungsform sieht vor, dass die Messelektroden 21-26 als Kammelektroden oder interdigitale Elektroden ausgebildet sind. Die Messelektroden 21-26 sind einander paarweise zugeordnet und weisen eine Kammstruktur auf, wobei die Zähne einander zugeordneter Messelektroden 21, 22; 23, 24; 25, 26 ineinander greifen. Wie in **Fig. 7 und Fig. 8** dargestellt, können Kammelektroden sowohl für eine Anordnung mit einem **(****Fig. 7****)** als auch mit mehreren Paaren von Messelektroden 21, 22; 23, 24; 25, 26 verwendet werden.

Je nach Anwendungsfall ist es auch möglich, unterschiedlich große Messelektroden 21-26 vorzusehen, um eine besonders vorteilhafte Bestimmung des Füllstandes F im Behälter 1 zu ermöglichen. Besonders vorteilhaft ist die Verwendung von parallelogrammförmigen bzw. dreieckigen Messelektroden 21-26, bei denen die Elektroden durch Trennbereiche 27 voneinander getrennt sind, die zur Vorschubrichtung V des Kolbens bzw. der Längsachse des Behälters 1 im Winkel verlaufen, beispielsweise in einem Winkel von 45°. Bei einer solchen Anordnung ergibt sich ein fließender Übergang, sodass eine besonders genaue Bestimmung des Füllstandes F möglich wird. Die **Fig. 9a bis 12d** zeigen vier unterschiedliche Ausführungsformen mit Trennbereichen 27 zwischen den Messelektroden 21-26, die im Winkel zur Vorschubrichtung V stehen. Weiters ist bei diesen Ausführungsformen ein achsparalleler Trennbereich 28 vorgesehen, der jeweils einander zugeordnete Paare von Messelektroden 21, 22; 23, 24; 25, 26 voneinander trennt.

Mit allen solchen Elektrodenanordnungen ist es möglich, aufgrund der Kapazität zwischen den Messelektroden 21-26 auf den Füllstand F des Behälters 1 zu schließen. Um eine möglichst präzise Messung der einzelnen Kapazitäten C₁, C₂, C₃ zu ermöglichen und damit einen Rückschluss auf den Füllstand F des Behälters 1 ziehen zu können, sieht die Erfindung bei einer Abgabevorrichtung vor, eine elektrische Abschirmung 3 für elektrische Felder außerhalb der Messelektroden 21-26 mantelförmig um den Behälter 1 anzuordnen. In **Fig. 13** ist ein Schnitt durch den Behälter 1 dargestellt, der die Abschirmung 3, die Messelektroden 21, 22, die Wand des Behälters und die Flüssigkeit 12 im Inneren des Behälters 1 darstellt. Die Abschirmung 3 bewirkt, dass die zwischen den Elektroden 21, 22 gemessene Kapazität nicht oder nur in vernachlässigbarem Ausmaß verfälscht wird, wenn eine Person die Abgabevorrichtung 100 berührt bzw. ihr nahekommt und dadurch die an den Messelektroden 21, 22 herrschenden elektrischen Feldbedingungen verändert. In einem ersten Ausführungsbeispiel der Erfindung ist die Abschirmung 3 als Folie aus elektrisch leitfähigem Material, beispielsweise als Kupferfolie mit einer Dicke von 50 µm ausgebildet, die mantelförmig um den Behälter 1 und die an diesem anliegenden Messelektroden 21, 22 gewickelt ist. Die Messelektroden 21, 22 und die Abschirmung 3 sind voneinander getrennt und nicht elektrisch leitend miteinander verbunden. Die Abschirmung 3 dient zur Unterdrückung des Einflusses von äußeren Beeinflussungen, z.B. Änderungen der Permittivität sowie elektrischen Feldern im unmittelbaren Außenbereich der Messelektroden 21, 22. Die Abschirmung 3 umgibt sowohl die Messelektroden 21, 22 als auch den Behälter 1 und befindet sich vorteilhafterweise nicht zwischen den Messelektroden und dem Behälter 1. Insbesondere erweist sich eine radiale Beabstandung der Abschirmung 3 zu den Messelektroden als vorteilhaft. Weiters ist es nicht notwendig, die Abschirmung 3 als vollflächige elektrisch leitfähige Folie auszuführen, sondern es besteht auch die Möglichkeit die Abschirmung 3 in Form von auf einem nicht elektrisch leitfähigen Trägermaterial, wie z.B. einer nicht elektrisch leitfähigen Folie, angeordneten einzelnen Leiterbahnen zu realisieren.

**Fig. 13a** zeigt das Detail Z aus **Fig. 1** im Schnitt B-B von **Fig. 13****.** Deutlich zu erkennen ist - wenn auch maßstäblich nicht korrekt - die Anordnung der Wand des Behälters 1 gegenüber den Elektroden 21, 23, 25 sowie der Abschirmung 3. Die einzelnen Leiter 32-34 auf der Folie 3 sind im Schnitt dargestellt. Außerhalb der Abschirmung 3 befindet sich das Gehäuse der Abgabevorrichtung 100.

Alternativ ist es auch möglich, die Abschirmung 3 unmittelbar außerhalb der Außenwand der Abgabevorrichtung 100 und/oder außerhalb eines den Behälter 1 zumindest teilweise umschließenden Trägers 31 anzuordnen. Der Träger 31 dient in diesem Fall gleichzeitig zur radialen Beabstandung zwischen der Abschirmung 3 und den Messelektroden 21,22

Zur Bestimmung des momentanen Füllstandes F der Flüssigkeit 12 in Behälter 1 wird zunächst die vorhandene Kapazität zwischen den Messelektroden 21, 22 bestimmt. In **Fig. 14** ist eine Messanordnung zur Bestimmung der Kapazität eines einzigen Paars von Messelektroden 21, 22 dargestellt. **Fig. 15** zeigt eine Messanordnung bei der Verwendung mehrerer Paare von Messelektroden 21-26. In **Fig. 14** **und** **15** ist jeweils eine Recheneinheit 6 in Form eines Mikrocontrollers vorgesehen, dem eine bzw. drei Kapazitätsmesseinrichtungen 41, 42, 43 vorgeschaltet sind. Jedem Paar von Messelektroden 21-26 ist jeweils eine der in **Fig. 15** dargestellten Kapazitätsmesseinrichtungen 41, 42, 43 zugeordnet. Die Messelektroden 21-26 sind jeweils mit den Anschlüssen der Kapazitätsmesseinrichtungen 41, 42, 43 verbunden. Am Ausgang der Kapazitätsmesseinrichtungen 41, 42, 43 liegt jeweils ein der jeweiligen Kapazität des jeweiligen Elektrodenpaars entsprechender, diese repräsentierender bzw. zu dieser proportionaler Kapazitätsmesswert C₁, C₂, C₃ an, der an die Recheneinheit 6 übertragen wird. Aufgrund der einzelnen übertragenen Kapazitätsmesswerte C₁, C₂, C₃ ermittelt die Recheneinheit 6 aufgrund eines später beschriebenen Kalibriervorgangs einen Wert für den Füllstand F. Die Recheneinheit 6 hält diesen Wert an ihrem Ausgang zur Verfügung. Dieser Wert kann insbesondere auf Anfrage über eine der Recheneinheit 6 nachgeschaltete Antenne 62 an ein (nicht dargestelltes) externes Datenkommunikationsgerät übertragen werden.

Selbstverständlich kann die Zahl der verwendeten Paare von Messelektroden 21-26 an die Anforderungen an die Genauigkeit der Messung angepasst werden. Insbesondere ist es auch möglich, ein einziges Paar von Messelektroden 21, 22 zu verwenden und nur den zwischen diesen Messelektroden 21, 22 ermittelten Kapazitätsmesswert C₁ zur Bestimmung des Füllstands F heranzuziehen. **(****Fig. 15****)**

Der Recheneinheit 6 ist ein Kommunikationscontroller 61 nachgeschaltet, der an eine Antenne 62 angeschlossen ist. Der Kommunikationscontroller 61 ermöglicht die Übertragung des ermittelten Füllstandes F an ein externes Datenkommunikationsgerät. Alternativ zur drahtlosen Übertragung füllstandsrelevanter Daten an ein externes Datenkommunikationsgerät ist selbstverständlich auch eine drahtgebundene Übertragung nach dem Stand der Technik, wie z.B. USB, möglich. Darüber hinaus kann auch noch vorgesehen sein, dass das externe Datenkommunikationsgerät über die Antenne 62 elektrische Energie an den Kommunikationscontroller 61, die Recheneinheit 6, sowie an die Kapazitätsmesseinrichtungen 41-43 überträgt, sodass die gesamte in **Fig. 14** **bzw.** **Fig. 15** dargestellte Schaltung ohne separate Energieversorgung auskommt.

Im Folgenden wird die konkrete Ermittlung des Füllstandes F der Flüssigkeit 12 im Behälter 1 anhand der ermittelten Kapazitätsmesswerte C₁, C₂, C₃ näher dargestellt. In **Fig. 17** ist jeweils die Abhängigkeit der einzelnen Kapazitätsmesswerte C₁, C₂, C₃ vom Füllstand F bei der in **Fig. 5** dargestellten Ausführungsform eines erfindungsgemäßen Behälters 1 schematisch dargestellt. Am Beginn des Entleervorgangs des Behälters 1 befindet sich zunächst ausschließlich die Flüssigkeit 12 zwischen den Messelektroden 21-26. Im Zuge der Entleerung gelangt der Kolben 13 zunächst in den Zwischenbereich zwischen die Messelektroden 21, 22 des ersten Messelektrodenpaars, sodass sich aufgrund der geringeren Permittivität des Kolbens 13 gegenüber der Flüssigkeit 12 ein kontinuierliches Absinken des Kapazitätsmesswertes C₁ des ersten Messelektrodenpaars zu beobachten ist. Nachdem der Kolben 13 durch den Zwischenbereich zwischen den Messelektroden 21, 22 des ersten Messelektrodenpaars geschoben wurde, befindet sich zwischen den beiden Messelektroden 21, 22 des ersten Messelektrodenpaars Luft 15. Aufgrund der noch geringeren Permittivität der Luft zwischen den beiden Messelektroden 21, 22 des ersten Messelektrodenpaars sinkt der zwischen diesen Messelektroden 21, 22 gemessene Kapazitätsmesswert C₁ noch weiter ab. Ein ähnliches Verhalten ist auch für die Kapazitätsmesswerte C₂, C₃ zwischen den Messelektroden 23-26 des zweiten und dritten Messelektrodenpaars bei Entleerung des Behälters 1 zu bemerken.

In einer besonderen Ausführungsform der Erfindung kann die Summe Cₛᵤₘ der einzelnen Kapazitätsmesswerte C₁, C₂, C₃ zur Bestimmung des Füllstands F herangezogen werden. Durch Ermittlung einer Kalibrierkurve kann für eine Anzahl von verschiedenen Füllständen jeweils die zugehörige Summe Cₛᵤₘ der einzelnen Kapazitätsmesswerte C₁, C₂, C₃ ermittelt werden, wobei jedem Füllstand F jeweils eine Summe Cₛᵤₘ zugeordnet wird. Die so erstellten einzelnen Datensätze umfassend jeweils einen Kapazitätsmesswert Cₛᵤₘ und einen Füllstand F werden in einem Kalibrierspeicher in der Recheneinheit 6 abgelegt. Alternativ ist es auch möglich, die einzelnen Kalibrier-Datensätze umfassend jeweils einen Kapazitätsmesswert Cₛᵤₘ und einen Füllstand F außerhalb der Abgabevorrichtung, beispielsweise in einem externen Datenkommunikationsgerät, für die spätere Bestimmung des Füllstandes bereit zu halten.

Nach der Messung und Bestimmung der einzelnen Kapazitätsmesswerte C₁, C₂, C₃ wird deren Summe Cₛᵤₘ ermittelt und mit den einzelnen im Kalibrierspeicher abgespeicherten Summen Cₛᵤₘ verglichen. Es wird dasjenige Paar von Elektroden ausgewählt, dessen zugehörige Summe Cₛᵤₘ mit der Summe der ermittelten Kapazitätsmesswerte C₁, C₂, C₃ am besten übereinstimmt. Der der am besten übereinstimmenden Summe Cₛᵤₘ jeweils zugeordnete Füllstand wird als Füllstand F des Behälters 1 angesehen, die Recheneinheit 6 hält diesen Füllstand F an ihrem Ausgang zur Verfügung und gibt den Füllstand F, wie vorstehend beschrieben, auf Anfrage über eine Antenne 62 oder selbständig ohne eine solche Anfrage an ein externes Datenkommunikationsgerät ab.

Die Praxis zeigt, offenbar aufgrund komplexer kapazitiver Verkopplungsphänomene der Messelektroden 21-26 untereinander auch stark abweichende Verläufe der gemessenen Kapazitäten C₁, C₂, C₃ in Abhängigkeit vom Füllstand F, die deutlich von den in **Fig. 17** dargestellten, theoretisch zu erwartenden Verläufen abweichen. Die messbaren Kurvenverläufe sind jedoch sehr gut reproduzierbar und zeigen für jede Kapazität C₁, C₂, C₃ unterschiedliche Steilheiten in unterschiedlichen Kurvenabschnitten bzw. Füllstandsbereichen, wobei entgegen den theoretischen Erwartungen die größte Steilheit des Kurvenverlaufs bzw. die größte Kapazitätsveränderung nicht zwangsläufig zwischen denjenigen Messelektroden 21-26 auftritt zwischen denen sich gerade der Flüssigkeitsspiegel befindet. Da größere Kurvensteilheit aber bessere Messauflösung/Messgenauigkeit bedeutet, kann für die Berechnung des Füllstands alternativ zur Bildung einer einfachen Summe der drei Einzel-Kapazitätsmesswerte eine gewichtete Summe verwendet werden, wobei für jeden der drei Summanden in jedem Kurvenabschnitt separat ein eigenes Gewicht im Zuge der Kalibrierung ermittelt wird.

Um eine Umrechnung zwischen einzelnen Kapazitäten C₁, C₂, C₃ und einem Füllstand F zu erreichen, wird eine Kalibrierung vorgenommen, bei der der mit dem Medikament gefüllte Behälter 1 oder ein baugleicher Referenzbehälter entleert wird. Während der Entleerung werden jeweils der Füllstand F sowie die einzelnen Kapazitäten C₁, C₂, C₃ ermittelt. Für jeden der bei der Entleerung eingenommenen Füllstände F stehen somit jeweils einzelne Kapazitätswerte C₁, C₂, C₃ zur Verfügung. Im vorliegenden Ausführungsbeispiel werden 30 äquidistande Füllstände F bei der Entleerung eingenommen, wobei der Ausgangszustand mit 1 und der vollständig entleerte Zustand mit 0 bezeichnet wird. Die Kapazitätswerte C₁, C₂, C₃ werden jeweils in einem Referenzvektor V_{ref} abgespeichert, der dem jeweiligen Füllstand F sowie den jeweiligen Gewichten a, b, c zugeordnet wird. Für jeden Füllstand F steht somit ein Referenzvektor V_{ref} zur Verfügung. Die Gewichte werden durch Optimierung derart festgelegt, dass die gewichtete Summe a . C₁ + b . C₂ + c . C₃ eine lineare Annäherung für den Füllstand F darstellt.

Will man nun den tatsächlichen Füllstand F anhand von durch Messung ermittelten Kapazitätswerten C₁, C₂, C₃ bestimmen, so kann dies aufgrund der während der Kalibrierung ermittelten Gewichte vorgenommen werden, wobei für jede Messung jeweils so viele Gewichte zur Verfügung stehen, wie Kapazitätswerte C₁, C₂, C₃ ermittelt wurden. Zunächst wird aufgrund der ermittelten bzw. gemessenen Kapazitätswerte C₁, C₂, C₃ ein Vektor Vₘₑₛₛ [C₁, C₂, C₃] erstellt, der die Kapazitätswerte C₁, C₂, C₃ als Komponenten aufweist. Anschließend wird der Vektor Vₘₑₛₛ mit den ermittelten Referenzvektoren V_{ref} verglichen und derjenige Referenzvektor gesucht, der zum Vektor Vₘₑₛₛ den geringsten Abstand aufweist. Im vorliegenden Ausführungsbeispiel wird als Abstandsmaß der Euklidische Abstand verwendet. Anschließend werden diejenigen Referenzvektoren V_{ref} ermittelt, der den jeweils nächstkleinsten Abstand zum Vektor Vₘₑₛₛ aufweisen. Es wird eine interpolierende Funktion, beispielsweise eine lineare Interpolationsfunktion bestimmt, die bei Anwendung auf die durch Kalibrierung ermittelten Referenzvektoren V_{ref} den jeweils diesen zugeordneten Füllstand F zurückliefert. Die Kapazitätswerte C₁, C₂, C₃ werden in die Interpolationsfunktion eingesetzt und man erhält einen gemittelten Füllstandswert.

Die Antenne 62 kann aus Platzgründen vorteilhafterweise außen auf der Abschirmung 3 angeordnet sein. Um eine vorteilhafte Kombination zu gewährleisten, weist die Abschirmung 3 eine Folie 31 aus einem elektrisch und magnetisch nicht leitfähigen Material, wie z.B. Kunststoff, auf. Auf der Folie 31, dargestellt in **Fig. 16****,** sind Leiter 32-34 in Form von Leiterbahnen aufgebracht. Wenn die Leiter 32-34 auf der Folie 31 derart ausgebildet sind, dass keine großflächig geschlossenen Leiterschleifen existieren, in denen sich Wirbelströme ausbilden können, werden die vom externen Datenkommunikationsgerät abgegebenen Magnetfelder von der Abschirmung 3 nicht maßgeblich beeinflusst und können von der Antenne 62 empfangen werden. Weiters ist es dadurch auch möglich, Energie in Form elektromagnetischer Wellen an die Antenne 62 zu übertragen, die ausreicht, um an die Antenne angeschlossene elektrische Bauteile ausreichend mit Energie zu versorgen.

Sofern eine zusätzliche Genauigkeit bei der Bestimmung des Füllstandes F im Inneren des Behälters 1 erforderlich ist, kann vorgesehen sein, dass ein Messwert für den Füllstand F dann invalidiert bzw. für ungültig erklärt wird, wenn das elektrische Feld im Außenbereich des Behälters 1, z.B. durch Berührungen bzw. das Nahekommen elektrisch leitfähiger Körper oder Körper mit hoher dielektrischer Permittivität verfälscht wurde.

Die Abschirmung 3 weist eine elektrisch und magnetisch nicht leitfähige Folie 31 auf, auf der eine Vielzahl von Leitern 32, 33, 34 durch Beschichtung ausgebildet ist. Die Folie 31 besteht im vorliegenden Ausführungsbeispiel aus biegsamem Kunststoff. Die Leiterbahnen weisen eine Schichtdicke von ca. 50 µm und eine Breite von etwa 1000 µm auf. Vorteilhaft sind Breiten der Leiter 32-34 zwischen 100 µm und 3000 µm.

Um die Ausbildung von Wirbelströmen und dadurch eine Beeinträchtigungen einer auf induktiver Kopplung beruhenden Kommunikation, insbesondere NFC-Kommunikation, zu vermeiden, kann die Breite der Leiter 32-34 auf weniger als 3 mm beschränkt werden. Darüber hinaus können die Leiter 32-34, wie in **Fig. 16** dargestellt, schleifenfrei, d.h. frei von geschlossenen Leiterschleifen, ausgebildet sein, d.h. keine geschlossenen Schleifen umfassen, um die Ausbildung von Wirbelströmen in hinreichendem Maße zu verhindern und eine Beeinträchtigung einer NFC-Kommunikation zu vermeiden, gleichzeitig jedoch auch kapazitive Beeinflussungen der innerhalb der Abschirmung 3 befindlichen Messelektroden 21-26 zu vermeiden.

In diesem besonderen Ausführungsbeispiel der Erfindung sind deshalb zwei der drei Leiter 32, 33 als ineinandergreifende Kammleiter 32, 33 ausgebildet, der dritte Leiter 34 verläuft mäanderförmig zwischen den beiden Kammleitern 32, 33. Neben diesem Ausführungsbeispiel gibt es selbstverständlich auch noch eine Vielzahl weiterer Ausführungsbeispiele der schleifenfreien Anordnung mehrerer miteinander nicht elektrisch verbundenen Leiterbahnen bzw. Elektroden auf der Oberfläche einer Folie 31 oder im Inneren bzw. zwischen einzelnen Lagen einer mehrlagig aufgebauten Folie. Auch können sowohl Vorderseite als auch Rückseite der Folie 31 mit Leitern 32-34 bedruckt sein. Alternativ können auch mehrere mäanderförmige Leiter 34 nebeneinander zwischen den Kammleitern 32, 33 angeordnet werden oder mehrere Leiter 34 spiralenförmig auf der Folie 31 angeordnet sein.

Zwei Leiterbahnen, nämlich einer der beiden Kammleiter 33 sowie der mäanderförmige Leiter 34, werden als Berührungssensor 5 verwendet. Der zweite Kammleiter 32 wird auf ein vorgegebenes Massepotential gelegt und dient als elektrische Abschirmung. Berührt eine Person die Abschirmung 3 oder kommt die Person der Abschirmung 3 nahe, so verändert sich aufgrund der Änderung der Permittivität der Umgebung die Kapazität zwischen den Leitern 33, 34 des Berührungssensors 5. Die Veränderung dieser Kapazität zwischen den Leitern 33, 34 kann mittels einer weiteren Kapazitätsmesseinrichtung 44 bestimmt werden, die Leiter 33, 34 der Abschirmung 3 bzw. des Berührungssensors 5 sind an die Messanschlüsse der weiteren Kapazitätsmesseinrichtung 44 angeschlossen. Diese weitere Kapazitätsmesseinrichtung 44 bestimmt einen weiteren Kapazitätswert C' und leitet diesen, wie in **Fig. 18** **bzw.** **Fig. 19** dargestellt, an die Recheneinheit 6 weiter. Übersteigt die Veränderung des ermittelten weiteren Kapazitätsmesswertes C' einen vorgegebenen Schwellenwert T, so wird angenommen, dass der aufgrund der Kapazitätsmesswerte C₁, C₂, C₃ ermittelte Füllstand F aufgrund der Berührung fehlerhaft ist. Der ermittelte Füllstand F wird invalidiert.

Im vorliegenden besonderen Ausführungsbeispiel der Erfindung wird eine Abschirmung 3 verwendet, die zugleich als Berührungsdetektion 5 fungiert und aus dem Kammleiter 32 und dem mäanderförmig verlaufenden Leiter 34 besteht. Aus physikalischer bzw. funktioneller Sicht sind elektrische Schirmung 3 und Berührungsdetektion 5 jedoch zwei völlig getrennte und unterschiedliche Einheiten, die sich durch die in **Fig. 16** dargestellte konkrete Anordnung besonders vorteilhaft, nämlich in einer Ebene durch Drucken herstellbar, realisieren lässt. Diese funktionelle Trennung von elektrischer Schirmung 3 und Berührungsdetektion 5 ist selbstverständlich ohne weiteres möglich. Nur zur einfacheren Darstellung wurden die in einer Ebene der Folie 31 liegenden Leiter 32, 33, 34 der Schirmung 3 bzw. der Berührungsdetektion 5 in den **Fig. 14****,** **15****,** **18, 19** nebeneinander dargestellt.

Eine alternative Ausführungsform der Erfindung ermöglicht die Entnahme des Behälters 1 aus der Abgabevorrichtung 100 bei einem Austausch. Außerhalb des Behälters 1 zwischen dem Behälter 1 und der Abschirmung 3 ist ein nicht dargestellter Träger angeordnet. Auf diesem befinden sich Messelektroden 21-26. Der Träger liegt am Behälter 1 an und ist vorteilhafterweise durch einen Teil des Gehäuses der Abgabevorrichtung 100 ausgebildet. Die Messelektroden 21-26 sind auf der am Behälter 1 anliegenden Wand des Trägers angeordnet. Das Gehäuse der Abgabevorrichtung 100 lässt sich öffnen und der Behälter 1 kann aus dem Gehäuse der Abgabevorrichtung 100 entfernt werden. Der Träger bildet einen Teil der Abgabevorrichtung 100.

Vorteilhafterweise können der Kommunikaitonscontroller 61, die Recheneinheit 6, die Kapazitätsmesseinrichtungen 41-44 sowie die Antenne 62 auf der Folie 31 angeordnet sein.

Eine weitere bevorzugte Ausführungsform der Erfindung, die in **Fig. 20** dargestellt ist, zeigt eine Abgabevorrichtung mit einer Abdeckkappe 9. Diese Ausführungsform entspricht im wesentlichen der zuvor dargestellten Ausführungsform, wobei im Folgenden lediglich die Unterschiede zur vorstehenden Ausführungsform näher dargestellt werden.

Die Abdeckkappe 9 ist mittels eines oder mehrerer Rastelemente 109 lösbar mit dem Trägerkörper 200 der Abgabevorrichtung verbunden. Die Abdeckkappe 9 umgibt den Behälter 1 sowie die auf dem Behälter 1 angeordneten Messelektroden 21-26 mantelförmig. Im Gegensatz zur vorherigen Ausführungsform sind die Abschirmung 3 und der Berührungssensor 5 im Körper der Abdeckkappe 9 angeordnet. Sofern die Abdeckkappe 9 auf den Trägerkörper 200 aufgesetzt ist, werden die Messelektroden 21-26 durch die Abschirmung 3 abgeschirmt. Die Abschirmung 3 ist in der vorliegenden Ausführungsform im Körper der Abdeckkappe 9 eingegossen und wird von diesem allseitig umschlossen. Wie auch bei der vorliegenden Ausführungsform ist die Abschirmung 3 auf einer Folie angeordnet, die von der Abdeckkappe 9 allseitig umgeben bzw. umschlossen ist. Im vorliegenden Fall umgibt die Abschirmung 3 die Messelektroden 21-26 im Endbereich der Abdeckkappe 9, der der Injektionsnadel 103 zugewandt ist. Es ist alternativ auch möglich, dass die Folie und/oder die Abschirmung 3 außen an der Abdeckkappe 9 angeordnet ist.

In diesem Endbereich weist die Abdeckkappe 9 eine Ausnehmung 99 als Durchlass für die Injektionsnadel 103 auf. Alternativ ist es auch möglich, dass die Injektionsnadel 103 vollständig vom Gehäuse der Abdeckkappe 9 umgeben ist.

Bei einer zweiten Alternative ist die Abdeckkappe 9 in Form einer Hülse ausgebildet, die an dem Ende der Injektionsnadel 103 offen ist. Damit ist eine Injektion bzw. Verabreichung der Flüssigkeit auch bei aufgesetzter Abdeckkappe möglich ist. Es ist somit insgesamt nicht erforderlich, dass die Abdeckkappe die Injektionsnadel 103 oder den Behälter 1 von allen Seiten her abdeckt.

Um einen Blick auf die Sichtöffnungen 108 im Trägerkörper 200 der Abgabevorrichtung zu ermöglichen, weist die Kappe 9 an der den Sichtöffnungen vorgelagerten Position jeweils weitere Sichtöffnungen 98 auf.

**Fig. 21** zeigt die Abgabevorrichtung 100 von außen. In dieser Darstellung ist auch eine Anzeigeeinheit 90 zu sehen, die an die Recheneinheit 6 (**Fig. 23****,** **25**) angeschlossen ist und von dieser angesteuert wird. Die Anschlussleitungen der Anzeigeeinheit 90 verlaufen im Inneren der Abdeckkappe 9 außerhalb der Abschirmung 3.

**Fig. 22a****-d** zeigen eine mögliche Ausgestaltung der Elektroden 21-26. Die einzelnen Messelektroden 21-26 sind über elektrisch leitende Verbundungen mit Anschlusskontakten 211, 221, 231, 241, 251, 261 geführt. Diese Verbindungen sind vorteilhafterweise ebenso wie die Messelektroden 21-26 und die Anschlusskontakte 211, 221, 231, 241, 251, 261 als Leiterschichten auf dem Behälter 1 innen oder außen angeordnet.

Grundsätzlich ist es möglich, hier sämtliche der in den **Fig. 9a bis 12d** dargestellten Elektrodenformen heranzuziehen, wobei die jeweiligen Messelektroden 21-26 jeweils über eine der Elektrode 21-26 zugeordnete im Außenbereich des Behälters 1 verlaufende Leitung zu den behälterseitigen Anschlusskontakten 211, 221, 231, 241, 251, 261 geführt sind. Sofern, wie in **Fig. 9a****-d** dargestellt, nur zwei Messelektroden verwendet werden, sind auch nur zwei Anschlusskontakte 211, 221 vorhanden.

**Fig. 23** zeigt eine Schnittdarstellung entlang des Schnitts B-B aus **Fig. 20** und. Der Trägerkörper 200 der Abgabevorrichtung 100 weist für jeden der Anschlusskontakte 211, 221, 231, 241, 251, 261 jeweils eine Durchkontaktierung 212, 222, 232, 242, 252, 262 auf, die an die äußere Oberfläche des Trägerkörpers 200 geführt ist und von außen abgreifbar und elektrisch kontaktierbar angeordnet ist.

Wie auch aus **Fig. 25** ersichtlich, weist die Abdeckkappe 9 an ihrem offenen und dem Trägerkörper 200 zugewandten Endbereich eine Anzahl von Anschlusskontakten 91, 92, 93, 94, 95, 96 auf, die mit den Durchkontaktierungen 212, 222, 232, 242, 252, 262 elektrisch leitend verbindbar sind. Um Verwechslungen zu vermeiden, können die Rastelemente 109 derart ausgebildet sein, dass ein Einrasten nur in einer einzigen Position möglich ist, bei der auch jede der Durchkontaktierungen 212, 222, 232, 242, 252, 262 jeweils mit einem der Anschlusskontakte 91, 92, 93, 94, 95, 96 der Abdeckkappe 9 kontaktiert ist. Sämtliche Anschlusskontakte 211, 221, 231, 241, 251, 261 und Durchkontaktierungen 212, 222, 232, 242, 252, 262, sowie Anschlusskontakte 91, 92, 93, 94, 95, 96 der Abdeckkappe 9 sind aus elektrisch leitfähigem Material, insbesondere aus Kupfer, ausgebildet.

Jeder der Anschlusskontakte 91, 92, 93, 94, 95, 96 ist mit jeweils einem Anschluss einer Kapazitätsmesseinheit 41, 42, 43 verbunden, sodass die jeweilige Kapazitätsmesseinheit 41, 42, 43 jeweils die Kapazität zwischen jeweils zwei einander gegenüberliegend angeordneten Messelektroden 21, 22; 23, 24; 25, 26 ermittelt. Die Kapazitätsmesseinheiten 41, 42, 43 sind an die Recheneinheit 6 angeschlossen.

Die in **Fig. 23** dargestellte Antenne 62 ist im Außenbereich der Abschirmung 3 auf oder in der Kappe 9 angeordnet. Im vorliegenden Ausführungsbeispiel ist die Antenne 62 wie auch die Abschirmung 3 mit der Kappe 9 vergossen und vom Körper der Kappe 9 vollständig umgeben. Die Antenne 9 ist vorzugsweise auf einer Folie angeordnet, die außerhalb der Folie der Abschirmung 3 angeordnet ist, wobei die beiden Folien vollständig vom Körper der Kappe umgeben sind.

Die Antenne 62 ist an einen Kommunikationscontroller angeschlossen, der mit der Recheneinheit 6 verbunden ist. Zwei der drei Leiter 32, 33, 34 der Abschirmung 3 sind elektrisch leitend mit der weiteren Kapazitätsmesseinrichtung 44 verbunden, die an die Recheneinheit 6 angeschlossen ist.

Die Kapazitätsmesseinrichtungen 41, 42, 43, die weitere Kapazitätsmesseinrichtung 44, der Kommunikationscontroller 61 sowie gegebenenfalls noch eine weitere in den Fig. nicht dargestellte Batterie sind in einem gemeinsamen Gehäuse 60 integriert, das im Inneren der Abdeckkappe 9 angeordnet, vorzugsweise mit dieser vergossen ist.

**Fig. 24** zeigt einen Schnitt C-C normal zur Vorschubrichtung im Endbereich des Behälters 1. Hier ist zu erkennen, dass die in der Kappe 9 angeordnete Abschirmung 3 die Messelektroden 21-26 umgibt. Im übrigen wird auf die Beschreibung der **Fig. 13** verwiesen.

**Fig. 26** zeigt eine weitere Ausführungsform der Erfindung, die allerdings auf eine Abschirmung 3 verzichtet. Um dennoch eine akkurate Messung zu gewährleisten, wird eine vergrößerte die Abdeckkappe 9 vorgesehen, die einen Zugriff auf die Messelektroden 21-26 auf weniger als 10mm vermeidet. Bei dieser Ausführungsform ist mangels einer Abschirmung auch keine weitere Kapazitätsmesseinrichtung vorgesehen.

## Patentansprüche

1. Abgabevorrichtung (100) zur Abgabe von Flüssigkeiten (12), insbesondere von flüssigen Medikamenten an Personen, mit einem Füllstandsmesssystem mit einer wiederverwendbaren Abdeckkappe umfassend
- einen mit der Flüssigkeit (12) gefüllten Behälter (1), der an einem Ende eine Öffnung (11) zur Abgabe der Flüssigkeit (12) aufweist,
- zumindest ein Paar von im Außenbereich, insbesondere anliegend an der Wand, des Behälters (1) einander gegenüberliegend angeordneten kapazitiven Messelektroden (21, 22) zur Bestimmung des Füllstandes (F) der Flüssigkeit (12) im Behälter (1),
- eine die Messelektroden (21, 22) und den Behälter (1) mantelförmig umgebende elektrische Abschirmung (3),bestehend aus auf einer Folie (31) aufgebrachten Leiterbahnen (32, 33, 34),
- einen auf der Folie (31) aufgebrachten Kommunikationscontroller (61)
- zumindest eine auf der Folie (31) aufgebrachte Kapazitätsmesseinrichtung (41) zur Ermittlung der Kapazität zwischen den Messelektroden (21, 22),
- eine auf der Folie (31) aufgebrachte Recheneinheit (6),
- eine Antenne (62) zur Übertragung von mit den Messelektroden (21, 22) ermittelten Messwerten oder daraus abgeleiteten Werten, insbesondere von Füllstandswerten (F),
- eine mittels einer lösbaren Verbindung an der Abgabevorrichtung (100) gekoppelte, die Messelektroden (21, 22) und den Behälter (1) mantelförmig umgebende Abdeckkappe (9), **dadurch gekennzeichnet, dass** die elektrische Abschirmung (3), die Kapazitätsmesseinrichtung (41), die Recheneinheit (6) und die Antenne (62) in der Abdeckkappe (9) integriert sind, wobei die Abschirmung (3) die Messelektroden (21, 22) und den Behälter (1) mantelförmig umgibt,
wobei die Abschirmung (3) in Form von auf einer nicht elektrisch leitfähigen Folie angeordneten einzelnen Leiterbahnen realisiert ist,
und dass die Abdeckkappe (9) eine Anzahl von elektrischen Kontakten aufweist, die jeweils mit einer der Messelektroden (21, 22) des Behälters (1) elektrisch leitend verbunden sind.

2. Abgabevorrichtung nach Anspruch 1, **dadurch gekennzeichnet, dass** die Leiter (32, 33, 34) schleifenfrei und/oder frei von geschlossenen Leiterschleifen ausgebildet sind und/oder dass die Leiter (32, 33, 34) eine Dicke von höchstens 3 mm, insbesondere von zwischen 50 µm und 150 µm, aufweisen.

3. Abgabevorrichtung nach einem der vorangegangenen Ansprüche, **dadurch gekennzeichnet, dass** auf der Folie (31) drei separate Leiter (32, 33, 34) ausgebildet sind, wobei der erste und der zweite Leiter (32, 33) als ineinandergreifende Kammleiter ausgebildet sind und der dritte Leiter (34) mäanderförmig ausgebildet zwischen den beiden Kammleitern (32, 33) liegt.

4. Abgabevorrichtung nach einem der vorangehenden Ansprüche, **dadurch gekennzeichnet, dass** die beiden gegenüberliegenden Messelektroden (21, 22) an eine Kapazitätsmesseinrichtung (41) angeschlossen sind, und dass vorzugsweise der von der Kapazitätsmesseinrichtung (41) ermittelte Kapazitätswert (C₁) einer Recheneinheit (6) zugeführt ist, die aufgrund des ermittelten Kapazitätswerts (C₁) mittels einer vorgegebenen abgespeicherten Kalibrierfunktion den Füllstand (F) der Flüssigkeit (12) im Behälter (1) bestimmt und an ihrem Ausgang zur Verfügung hält.

5. Abgabevorrichtung nach Anspruch 4, **dadurch gekennzeichnet, dass** einer der drei Leiter (32, 33, 34), insbesondere der als Kammleiter ausgebildete zweite Leiter (33), mit dem Massenanschluss der Kapazitätsmesseinrichtung (41) verbunden ist.

6. Abgabevorrichtung nach einem der vorangehenden Ansprüche, **gekennzeichnet durch** einen außerhalb oder im Bereich der Abschirmung (3) angeordneten, insbesondere kapazitiven, Berührungssensor (5), wobei vorzugsweise der Berührungssensor (5) den ersten Kammleiter (32) und den mäanderförmigen Leiter (34) der Abschirmung (3) als Sensorelektroden umfasst.

7. Abgabevorrichtung nach Anspruch 6,**dadurch gekennzeichnet, dass** die Sensorelektroden des Berührungssensors (5) an eine weitere Kapazitätsmesseinrichtung (44) angeschlossen sind, und dass vorzugsweise der von der weiteren Kapazitätsmesseinrichtung (44) ermittelte weitere Kapazitätswert (C') der Recheneinheit (6) zugeführt ist, und die Recheneinheit (6) für den Fall, dass der ermittelte weitere Kapazitätswert (C') einen vorgegebenen Schwellenwert (T) übersteigt, die Weiterleitung des von ihr ermittelten Füllstands (F) unterdrückt oder als ungültig kennzeichnet.

8. Abgabevorrichtung nach einem der vorangehenden Ansprüche, **dadurch gekennzeichnet, dass** am Behälter (1) eine Vielzahl von Paaren von zusätzlichen Messelektroden (23, 24; 25, 26) angeordnet sind, wobei insbesondere an jedes Paar von zusätzlichen Messelektroden (23, 24; 25, 26) jeweils eine zusätzliche dem Paar von zusätzlichen Messelektroden (23, 24; 25, 26) nachgeschaltete Kapazitätsmesseinrichtung (42, 43) vorgesehen ist, die den ermittelten Kapazitätswert (C₂, C₃) an die Recheneinheit (6) abgibt.

9. Abgabevorrichtung nach einem der vorangehenden Ansprüche, **dadurch gekennzeichnet, dass** die jeweils einander paarweise zugeordneten Messelektroden (21, 22; 23, 24; 25, 26) einander in Umfangsrichtung des Behälters (1), insbesondere diametral, gegenüberliegen und insbesondere in Richtung des Vorschubs des Kolbens (13) auf derselben Höhe liegen.

10. Abgabevorrichtung nach einem der vorangegangenen Ansprüche, **dadurch gekennzeichnet, dass** die Abdeckkappe (9) im Bereich der Öffnung (11) des Behälters (1) eine durchgehende Ausnehmung (99) zur Abgabe von Flüssigkeit durch die Abdeckkappe (9) hindurch aufweist.

11. Abgabevorrichtung nach einem der vorangegangenen Ansprüche, **dadurch gekennzeichnet, dass** die Abdeckkappe (9) eine Batterie enthält, die an die Kapazitätsmesseinrichtung (41, 42, 43), die weitere Kapazitätsmesseinrichtung (44),die Recheneinheit (6) und den Kommunikationscontroller (61) angeschlossen ist und diese mit elektrischer Energie versorgt und
dass die Batterie, die Kapazitätsmesseinrichtung (41, 42, 43) und der Kommunikationscontroller (61) in einem gemeinsamen Gehäuse (60) integriert sind.

## Claims

1. A dispensing device (100) for dispensing liquids (12), in particular liquid medicaments to persons, with a fill level measuring system with a reusable cover cap comprising
- a container (1) filled with the liquid (12) and having at one end an opening (11) for dispensing the liquid (12),
- at least one pair of capacitive measuring electrodes (21, 22) arranged opposite to one another in the outer region of the container (1), in particular adjacent to the wall, for determining the fill level (F) of the liquid (12) in the container (1),
- an electrical shield (3) which surrounds the measuring electrodes (21, 22) and the container (1) in a jacket-like manner, consisting of conductive paths (32, 33, 34) applied to a film (31),
- a communication controller (61) applied to the film (31),
- at least one capacitance measuring device (41) applied to the foil (31) for determining the capacitance between the measuring electrodes (21, 22),
- a computing unit (6) applied to the film (31),
- an antenna (62) for transmitting measured values determined by the measuring electrodes (21, 22) or values derived therefrom, in particular fill level values (F),
- a jacket-like covering cap (9) which is coupled to the dispensing device (100) by means of a detachable connection and which surrounds the measuring electrodes (21, 22) and the container (1) in a jacket-like manner,
**characterised in that** the electrical shield (3), the capacitance measuring device (41), the computing unit (6) and the antenna (62) are integrated into the covering cap (9), wherein the shield (3) surrounds the measuring electrodes (21, 22) and the container (1) in a jacket-like manner,
wherein the shield (3) is realized in the form of individual conductive paths arranged on a non-electrically conductive film,
and that the cover cap (9) has a number of electrical contacts which are each electrically conductively connected to one of the measuring electrodes (21, 22) of the container (1).

2. The dispensing device according to claim 1, **characterised in that** the conductors (32, 33, 34) are embodied to be loop-free and/or free of closed conductor loops and/or that the conductors (32, 33, 34) have a thickness of at most 3 mm, in particular of between 50 µm and 150 µm.

3. The dispensing device according to one of the preceding claims, **characterised in that** three separate conductors (32, 33, 34) are embodied on the film (31), wherein the first and the second conductor (32, 33) are embodied as interengaging comb conductors and the third conductor (34) is embodied to be meandering between the two comb conductors (32, 33).

4. The dispensing device according to one of the preceding claims, **characterised in that** the two opposing measuring electrodes (21, 22) are connected to a capacitance measuring device (41), and that preferably the capacitance value (C₁) determined by the capacitance measuring device (41) is fed to a computing unit (6), which determines the fill level (F) of the liquid (12) in the container (1) based on the determined capacitance value (C₁) by means of a predetermined stored calibration function, and makes the output thereof available.

5. The dispensing device according to claim 4, **characterised in that** one of the three conductors (32, 33, 34), in particular the second conductor (33) embodied as a comb conductor, is connected to the earth connection of the capacitance measuring device (41).

6. The dispensing device according to one of the preceding claims, **characterised by** a contact sensor (5), preferably a capacitative contact sensor (5), arranged outside of or in the region of the shield (3), wherein the contact sensor (5) preferably comprises the first comb conductor (32) and the meandering conductor (34) of the shield (3) as sensor electrodes.

7. The dispensing device according to claim 6, **characterised in that** the sensor electrodes of the contact sensor (5) are connected to an additional capacitance measuring device (44), and that the additional capacitance value (C') determined by the additional capacitance measuring device (44) is preferably fed to the computing unit (6), and in the event that the determined additional capacitance value (C') exceeds a predetermined threshold value (T), the computing unit (6) suppresses the transmission of the fill level (F) determined thereby or marks same as invalid.

8. The dispensing device according to one of the preceding claims, **characterised in that** a plurality of pairs of additional measuring electrodes (23, 24; 25, 26) are arranged on the container (1), wherein in particular to each pair of additional measuring electrodes (23, 24; 25, 26) is provided in each case an additional capacitance measuring device (42, 43) connected downstream of the pair of additional measuring electrodes (23, 24; 25, 26), which outputs the determined capacitance values (C₂, C₃) to the computing unit (6).

9. The dispensing device according to one of the preceding claims, **characterised in that** the respectively paired measuring electrodes (21, 22; 23, 24; 25, 26) arranged pairwise with one another in the circumferential direction of the container (1), in particular diametrically opposite, and in particular in the direction of the feed of the piston (13), are at the same height.

10. The dispensing device according to one of the preceding claims, **characterised in that** the cover cap (9) in the region of the opening (11) of the container (1) has a piercing recess (99) for dispensing liquid through the cover cap (9).

11. The dispensing device according to one of the preceding claims, **characterised in that** the covering cap (9) contains a battery which is connected to the capacitance measuring device (41, 42, 43), the additional capacitance measuring device (44), the computing unit (6) and the communication controller (61), and supplies all of same with electric power and,
**in that** the battery, the capacitance measuring device (41, 42, 43) and the communication controller (61) are integrated into a common housing (60).

## Revendications

1. Dispositif d'administration (100) pour l'administration de fluides (12), en particulier de médicaments fluides, à des personnes, comprenant un système de mesure de l'état de remplissage avec un capot de protection réutilisable, comprenant
- un récipient (1) rempli du fluide (12), qui présente, au niveau d'une extrémité, une ouverture (11) pour l'administration du fluide (12),
- au moins une paire d'électrodes de mesure capacitives (21, 22) disposées l'une en face de l'autre dans la zone externe, notamment adjacente à la paroi du récipient (1) destinées à déterminer l'état de remplissage (F) du fluide (12) dans le récipient (1),
- un blindage électrique (3), entourant à la manière d'une enveloppe les électrodes de mesure (21, 22) et le récipient (1), constitué de pistes conductrices (32, 33, 34) appliquées sur une feuille (31),
- un organe de commande de communication (61) appliqué sur la feuille (31),
- au moins un dispositif de mesure de capacité (41) appliqué sur la feuille (31) pour la détermination de la capacité entre les électrodes de mesure (21, 22),
- une unité arithmétique (6) appliquée sur la feuille (31),
- une antenne (62) pour la transmission de valeurs de mesure déterminées par les électrodes de mesure (21, 22) ou de valeurs qui en découlent, notamment de valeurs d'état de remplissage (F),
- un capot de protection (9) couplé au moyen d'une liaison détachable au dispositif d'administration (100) et entourant à la manière d'une enveloppe les électrodes de mesure (21, 22) et le récipient (1),
**caractérisé en ce que** le blindage électrique (3), le dispositif de mesure de capacité (41), l'unité arithmétique (6) et l'antenne (62) sont intégrés dans le capot de protection (9), dans lequel le blindage (3) entoure à la manière d'une enveloppe les électrodes de mesure (21, 22) et le récipient (1),
dans lequel le blindage (3) est réalisé sous la forme de pistes conductrices individuelles disposées sur une feuille non électroconductrice
et **en ce que** le capot de protection (9) présente un certain nombre de contacts électriques, qui sont chacun connectés de façon électroconductrice à une des électrodes de mesure (21, 22) du récipient (1).

2. Dispositif d'administration selon la revendication 1, **caractérisé en ce que** les conducteurs (32, 33, 34) sont formés sans boucle et/ou dépourvus de boucles conductrices fermées et/ou **en ce que** les conducteurs (32, 33, 34) présentent une épaisseur maximum de 3 mm, notamment entre 50 µm et 150 µm.

3. Dispositif d'administration selon une des revendications précédentes, **caractérisé en ce que** trois conducteurs séparés (32, 33, 34) sont formés sur la feuille (31), dans lequel les premier et deuxième conducteurs (32, 33) sont conçus sous forme de conducteurs en peigne s'engrenant l'un dans l'autre et le troisième conducteur (34) est conçu en forme de méandres et placé entre les deux conducteurs en peigne (32, 33).

4. Dispositif d'administration selon une des revendications précédentes, **caractérisé en ce que** les deux électrodes de mesure (21, 22) opposées sont raccordées à un dispositif de mesure de capacité (41) et **en ce que**, de préférence, la valeur de capacité (C₁) déterminée par le dispositif de mesure de capacité (41) est transférée à une unité arithmétique (6), qui, sur la base de la valeur de capacité (C₁) déterminée, détermine, au moyen d'une fonction d'étalonnage prédéfinie mémorisée, l'état de remplissage (F) du fluide (12) dans le récipient (1) et le tient à disposition au niveau de sa sortie.

5. Dispositif d'administration selon la revendication 4, **caractérisé en ce qu'**un des trois conducteurs (32, 33, 34), en particulier le deuxième conducteur (33) conçu comme un conducteur en peigne, est raccordé au raccordement à la masse du dispositif de mesure de capacité (41).

6. Dispositif d'administration selon une des revendications précédentes, **caractérisé par** un capteur de contact (5), notamment capacitif, disposé en dehors ou dans la zone du blindage (3), dans lequel, de préférence, le capteur de contact (5) comprend le premier conducteur en peigne (32) et le conducteur (34) en forme de méandres du blindage (3) comme électrodes de capteur.

7. Dispositif d'administration selon la revendication 6, **caractérisé en ce que** les électrodes de capteur du capteur de contact (5) sont raccordées à un autre dispositif de mesure de capacité (44) et **en ce que**, de préférence, l'autre valeur de capacité (C') déterminée par l'autre dispositif de mesure de capacité (44) est transférée à l'unité arithmétique (6) et l'unité arithmétique (6), au cas où l'autre valeur de capacité (C') déterminée dépasse une valeur seuil prédéfinie (T), supprime ou considère invalide la transmission de l'état de remplissage (F) qu'elle a déterminée.

8. Dispositif d'administration selon une des revendications précédentes, **caractérisé en ce que**, sur le récipient (1), est disposée une pluralité de paires d'électrodes de mesure supplémentaires (23, 24 ; 25, 26), dans lequel, en particulier, pour chaque paire d'électrodes de mesure supplémentaires (23, 24 ; 25, 26), respectivement, un dispositif de mesure de capacité (42, 43) supplémentaire est prévu en aval de la paire d'électrodes de mesure supplémentaires (23, 24 ; 25, 26), qui émet les valeurs de capacité (C₂, C₃) déterminées vers l'unité arithmétique (6).

9. Dispositif d'administration selon une des revendications précédentes, **caractérisé en ce que** les électrodes de mesure supplémentaires (21, 22 ; 23, 24 ; 25, 26) disposées respectivement par paire sont disposées opposées les unes aux autres dans la direction circonférentielle du récipient (1), notamment diamétralement opposées et, en particulier, dans la direction d'avance du piston (13) à la même hauteur.

10. Dispositif d'administration selon une des revendications précédentes, **caractérisé en ce que** le capot de protection (9) présente à travers lui, dans la zone de l'ouverture (11) du récipient (1) une cavité traversante (99) pour l'administration du fluide à travers le capot de protection (9).

11. Dispositif d'administration selon une des revendications précédentes, **caractérisé en ce que** le capot de protection (9) comprend une batterie, qui est raccordée au dispositif de mesure de capacité (41, 42, 43), à l'autre dispositif de mesure de capacité (44), à l'unité arithmétique (6) et à l'organe de commande de communication (61) et les alimente en énergie électrique et
**en ce que** la batterie, le dispositif de mesure de capacité (41, 42, 43) et l'organe de commande de communication (61) sont intégrés dans un boîtier commun (60).
